(19) Europäisches Patentamt
European Patent Office
Office européen des brevets

(11) Publication number: **0 089 730 B1**

(12) **EUROPEAN PATENT SPECIFICATION**

(45) Date of publication of patent specification: **27.05.87**

(51) Int. Cl.⁴: **C 07 D 277/48,** C 07 D 263/48, C 07 D 271/06, C 07 D 285/08, C 07 D 285/12, A 61 K 31/41

(21) Application number: **83200388.3**

(22) Date of filing: **22.03.83**

# (54) Guanidino heterocyclicphenylamidines, processes for their preparation and their pharmaceutical use.

(30) Priority: **24.03.82 IT 2035682**

(43) Date of publication of application: **28.09.83 Bulletin 83/39**

(45) Publication of the grant of the patent: **27.05.87 Bulletin 87/22**

(84) Designated Contracting States: **AT BE CH DE FR IT LI LU NL SE**

(56) References cited:
**EP-A-0 003 640**
**EP-A-0 014 057**
**EP-A-0 053 407**

(73) Proprietor: **ISTITUTO DE ANGELI S.p.A.**
**Via Serio, 15**
**I-20139 Milano (IT)**

(72) Inventor: **Bietti, Giuseppe**
**Via Lario 31**
**I-20159 Milan (IT)**
Inventor: **Cereda, Enzo**
**Via Romagnolo 2A**
**I-15057 Tortona (Alessandria) (IT)**
Inventor: **Donetti, Arturo**
**Viale Romagna 4**
**I-20133 Milano (IT)**
Inventor: **Del Soldato, Piero**
**Via E. Toti 22**
**I-20052 Monza (Milan) (IT)**
Inventor: **Giachetti, Antonio**
**Via Guerrini 3**
**I-20133 Milan (IT)**
Inventor: **Micheletti, Rosamaria**
**Via Metastasio 3**
**I-20123 Milano (IT)**

(74) Representative: **Appoloni, Romano et al**
**ING. BARZANO' & ZANARDO MILANO S.p.A. Via Borgonuovo 10**
**I-20121 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 089 730 B1

**Description**

The present invention relates to new pharmacologically active substituted guanidino heterocyclicphenylamidine compounds which are $H_2$- receptor blocking agents, which inhibit gastric acid secretion, and which are useful antiulcer agents.

Histamine antagonists have been studied for many years. In the last forty years, various substances, such a mepyramine, were found to antagonize certain effects ($H_1$) of histamine. These substances however had no effect on gastric acid secretion which is mediated by other histamine receptors ($H_2$) (Black, *et al,* Nature *236,* 385, 1972).

With the advent of cimetidine, preceded by two other compounds (burimamide and metiamide), a new type of antihistamines ($H_2$-blockers) has been discovered capable of antagonizing those responses (e.g. gastric acid secretion) which are unaffected by classical antihistamines ($H_1$-blockers).

More recently new $H_2$-receptor antagonists, i.e. ranitidine (Bradshaw, *et at,* Brit. J. Pharmacol. *66,* 464 P, 1979), tiotidine (P.O. Jellin, Life Sci., *25,* 2001, 1979) and BL.6341 (Cavanagh, *et al* Fed. Proc., *40,* 2652, 1981), have been reported to be more potent that cimetidine both as $H_2$-receptor antagonists "in vitro" and as inhibitors of gastric acid secretion "in vivo".

All these compounds share a common feature, namely a methylthioethyl side-chain ($CH_2SCH_2CH_2$) bearing neutral polar groups connecting basic or basic-substituted heterocyclic rings.

Other new $H_2$-receptor antagonists have been claimed in the EP—A—0003640 and EP—A—0014057 which are structurally reminiscent of the compounds object of the present invention.

In the EP—A—0053407 we have described a new type of $H_2$-receptor antagonists, i.e. imidazolylphenylamidines, which are potent $H_2$-receptor antagonists and inhibitors of gastric acid secretion. With respect to cimetidine, in these compounds the imidazole ring is retained, whereas the methylthioethyl side-chain is replaced by a phenyl ring connecting an amidino system.

According to the present invention there are provided compounds of general formula (I)

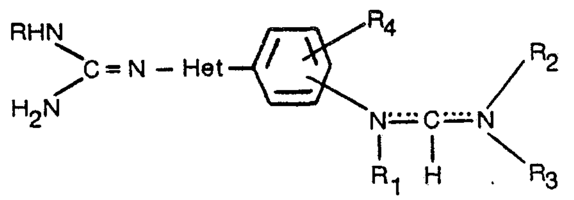

(I)

wherein R, $R_1$ and $R_2$, which may be the same or different, each represents a hydrogen atom or an alkyl group having 1 to 4 carbon atoms, $R_3$ represents a linear or branched $C_1$—$C_8$ alkyl group containing optionally heteroatoms such as oxygen sulfur or nitrogen atom, a linear or branched $C_3$—$C_5$ alkenyl group, a $C_3$—$C_4$ alkynyl group, a cyano group, a $C_3$—$C_6$ cycloalkyl or cycloaliphatic alkyl group, a norbornyl group, a phenyl group, an unsaturated six-membered heterocyclic ring, or $R_3$ and $R_2$ together with the adjacent nitrogen atom represent a saturated five or six-membered heterocyclic ring which may contain a further heteroatom, $R_4$ represents a hydrogen atom, an alkyl or alkoxy group both having 1 to 4 carbon atoms, or halogen atom, Het represents a substituted or unsubstituted five-membered heterocyclic ring containing two or three heteroatoms, and tautomers thereof and acid addition salts of the aforesaid compounds, the dotted line denoting that a double bond between the C atom and one of the N atoms is present, and that either $R_1$ or $R_2$ are not present, respectively.

For pharmaceutical use the acid addition salts referred to the above will be physiologically compatible acid addition salts. The term "acid addition salts" includes salts with inorganic or organic acids. Suitable physiologically compatible acid addition salts include, for example, salts formed with hydrochloric, nitric, sulphuric, maleic, fumaric, citric or tartaric acids.

It is to be understood that the dotted lines in the amidine radical in the formula (I) indicate two tautomeric forms (Ia) and (Ib)

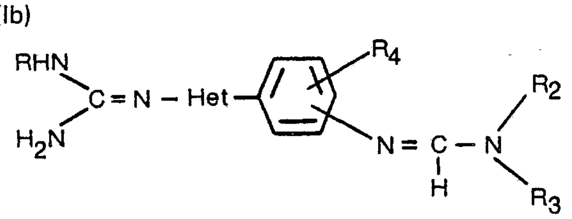

(Ia)

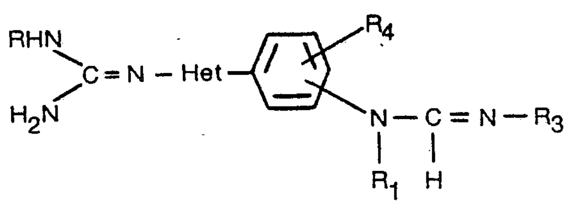

(Ib)

Although the double bonds in the other groups have been inserted in particular positions, other tautomeric forms are possible and the present invention includes such tautomeric forms within its scope, both in terms of the compounds of the invention and in terms of the manufacturing processes.

When in the compounds of formula (I) R, $R_1$ and/or $R_2$ represent alkyl groups these have 1 to 4 carbon atoms, in particular with the proviso that when $R_1$ is present, $R_2$ is absent and vice-verca; when $R_3$ represents a linear or branched alkyl group it contains from 1 to 8 carbon atoms which may optionally contain an oxygen atom, e.g. methoxyethyl or hydroxypropyl group, a sulfur atom, e.g. a methylthioethyl group, a nitrogen atom, e.g. cyanoethyl group; when $R_3$ is a linear or branched alkenyl group it contains from 3 to 5 carbon atoms; when $R_3$ represents an alkynyl group it contains 3 or 4 carbon atoms; when $R_3$ is a bicyclic group it is a norbornyl group; $R_3$ may be a phenyl group; when $R_3$ represents a cycloalkyl or cycloaliphatic alkyl group it contains from 3 to 6 carbon atoms; when $R_3$ represents a heterocyclic group, it is an unsaturated six-membered ring, e.g. pyridyl; when $R_3$ and $R_2$ together with the adjacent nitrogen atom represent a heterocylic group, this is a saturated five or six-membered ring which may contain a further heteroatom, e.g. pyrrolidine or morpholine; when $R_4$ represents an alkyl or alkoxy group having 1 to 4 carbon atoms it may, for example, be methyl or methoxy; when $R_4$ represents a halogen atom, it may, for example, be a chlorine atoms; when Het represents a substituted or unsubstituted heterocyclic group containing two or three heteroatoms, it may for example be e.g. a thiazole, oxazole, 1,3,4-thiadiazole, 1,2,4-thiadiazole, 1,2,4-oxadiazole ring. In the above mentioned formula (I) the amidine radical may be in the ortho, meta or para position of the benzene ring with respect to the Het group, and the group $R_4$ is in any position in the benzene ring.

Preferred compounds according to the present invention include those wherein the amidine radical is in the meta position of the benzene ring, R, $R_1$ and $R_4$ are hydrogen atoms, $R_2$ is absent, $R_3$ is methyl, ethyl, n-propyl, isopropyl, n-butyl, isobutyl, sec-butyl, neopentyl, n-hexyl, n-octyl, allyl, dimethylallyl, propargyl, cyclopropylmethyl, cyclohexyl, norbornyl, phenyl, pyridyl, hydroxypropyl, methoxyethyl, methylthioethyl or cyanoethyl group, Het is thiazolyl or 1,2,4-thiadiazolyl group and their physiologically compatible acid addition salts. Such compounds generally have better activity and are therefore preferred as antisecretory-antiulcer agents and for the treatment of disorders of the gastrointestinal tract.

The compounds of general formula (I) may, for example, be prepared by the following processes which constitute further features of the invention.

Process A

Reaction of a guanidinoheterocyclylphenylamine of formula (II)

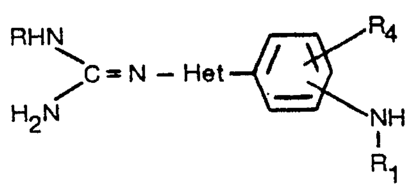

(II)

in which R, $R_1$, $R_4$ and Het are as hereinbefore defined, with a reactive derivative of a carboxamide of formula (III)

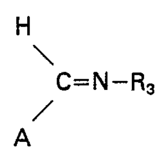

(III)

where $R_3$ is as hereinbefore defined and A represents a lower alkoxy group such as methoxy or ethoxy group. The reaction is generally effected at a temperature from 0° to 100°C, preferably from 20° to 60°C. The reaction is advantageously carried out in the presence of an inert organic solvent, for example alcohols having 1 to 3 carbon atoms such as methanol or ethanol, halogenated hydrocarbons such as dichloromethane, dioxane or acetone.

The compounds of formula (III) used as starting material in the above process may be prepared by conventional methods by reacting an amine of formula (IV)

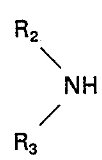

(IV)

wherein $R_2$ and $R_3$ are as above defined, with a compound of formula (V)

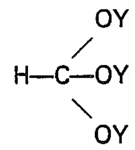

(V)

wherein Y is a lower alkyl group such as methyl or ethyl group in the presence of mineral acids.

Process B

Reaction of a N,N-disubstituted carboxamide dialkyl acetal of formula (VI)

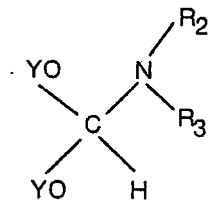

(VI)

in which $R_2$, $R_3$ and Y are as hereinbefore defined with an amine of formula (II). The process is carried out at a temperature from 20° to 80°C and by distilling the alcohol formed during the reaction.

Process C

Reaction of a N,N'-disubstituted amidine of formula (VIIa) or (VIIb)

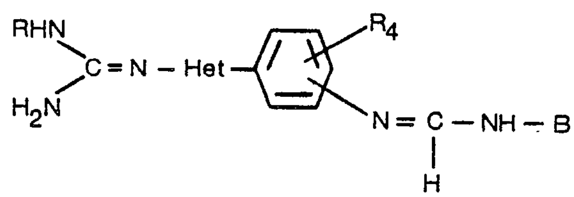

(VIIa)

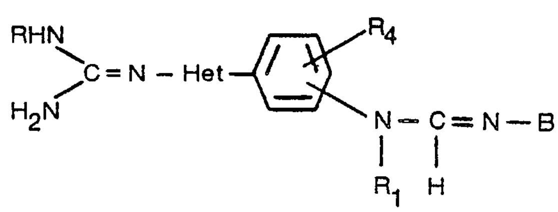

(VIIb)

wherein R, $R_1$, $R_4$ and Het are as hereinbefore defined and B represents a leaving group such as cyano, acetyl, carbethoxy or carbamyl group, with an amine of formula (IV).

The reaction is conveniently performed in the presence of water or of an inert organic solvent, for example lower alcohols, such as methanol or ethanol, formamide, dimethylformamide, dioxane or acetonitrile.

The reaction is generally carried out at a temperature from 10° to 50°C, preferably at room temperature. The compounds of formula (VIIa) and (VIIb) used as starting materials in the above process are obtained by methods that are known per se in the literature, for example by reacting an amine of formula (II) with a N-substituted alkyl imidate of formula (VIII)

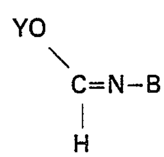

(VIII)

in which Y and B are as hereinbefore defined, or optionally, when in the compounds of formula (VIIa) and (VIIb) B represents a cyano group, the reaction may also be performed in a single step by reacting an amine of formula (II) with cyanamide in the presence of a compound of formula (V). The reaction is generally carried out in the presence of a suitable solvent such as a lower alcohol, ethers, ethyl acetate, acetonitrile or

dioxane, or without solvent at a temperature from 20° to 80°C. The compounds of formula (VIII) may be prepared by conventional methods.

The compounds of formula (I) prepared according to the processes A to C may optionally be converted with inorganic or organic acids into physiologically compatible acid addition salts, for example by conventional methods, such as by reacting the compounds as bases with a solution of the corresponding acid in a suitable solvent. Particularly preferred acids include for example hydrochloric, sulphuric, maleic and fumaric acid. The compounds of general formula (I) and their physiologically compatible acid addition salts are $H_2$-receptor blocking agents which inhibit the gastric acid secretion.

Particularly preferred compounds according to the present invention are the following:

N-Methyl-N'-[3-(2-guanidino-4-thiazolyl)phenyl] formamidine (Compound 1)
N-Ethyl-N'-[3-(2-guanidino-4-thiazolyl)phenyl] formamidine (Compound 2)
N-Propyl-N'-[3-(2-guanidino-4-thiazolyl)phenyl] formamidine (Compound 3)
N-Isopropyl-N'-[3-(2-guanidino-4-thiazolyl)phenyl] formamidine (Compound 4)
N-Butyl-N'-[3-(2-guanidino-4-thiazolyl)phenyl] formamidine (Compound 5)
N-sec.Butyl-N'-[3-(2-guanidino-4-thiazolyl)phenyl] formamidine (Compound 6)
N-Isobutyl-N'-[3-(2-guanidino-4-thiazolyl)phenyl] formamidine (Compound 7)
N-Allyl-N'-[3-(2-guanidino-4-thiazolyl)phenyl] formamidine (Compound 11)
N-Propargyl-N'-[3-(2-guanidino-4-thiazolyl)phenyl] formamidine (Compound 13)
N-(2-Methoxyethyl)-N'-[3-(2-guanidino-4-thiazolyl)phenyl] formamidine (Compound 17)
N-(3-Hydroxypropyl)-N'-[3-(2-guanidino-4-thiazolyl)phenyl] formamidine (Compound 20)
N-Ethyl-N'-[3-(5-guanidino-1,2,4-thiadiazolyl)phenyl] formamidine (Compound 45)

The antagonistic activity of compounds according to the invention on histamine $H_2$-receptors is demonstrated either "in vitro" or "in vivo" by their inhibition of the $H_2$-dependent biological effects, which include the histamine evoked position chronotropic effect and the histamine induced gastric secretion of acid respectively.

The inhibition of the positive chronotropic effect has been investigated in isolated guinea pig atria suspended in an organ bath (50 ml) containing oxygenated ($0_2$:95%—$CO_2$:5%) Krebs—Henseleit solution (pH 7.4) maintained at 32°C. The myocardial preparation, loaded with 1 g isometric tension, is allowed to stabilize 60 min., and myocardial contractions are recorded through an isometric lever connected to a strain-gauge coupler, and the instantaneous rate is monitored with a cardiotachometer, and a heatwriting pen recorder. After two control responses to histamine ($10^{-6}$g.ml$^{-1}$) the test compounds are added to the bath at the desired final concentration and left for 30 minutes before the atria are again challenged with histamine. The chronotropic response obtained in the presence of the antagonist is then compared to the control response to histamine and the percent reduction of the histamine $H_2$-evoked response is calculated. The average effective concentration ($EC_{50}$) of the $H_2$ antagonists is calculated by standard procedure according to Dr. Waud, Analysis of dose-response curves, in "Methods in Pharmacology" vol. 3 Smooth muscle, Ed Daniel E. E. Paton, M., Plenum Press, New York (1975); Ash and Schild, Br. J. Pharmacol. Chemother. *27,* 427—439, 1966. The following Table shows the obtained results.

TABLE 1
"In vitro" inhibitory activity in histamine induced tachycardia (guinea pig atria)

| Compound | $EC_{50} 10^{-7}M$ |
|---|---|
| 1 | 3.3 |
| 2 | 5.3 |
| 3 | 2.8 |
| 4 | 2.0 |
| 5 | 5.5 |
| 6 | 3.0 |
| 7 | 12.0 |
| 11 | 3.3 |
| 13 | 31.0 |
| 17 | 4.8 |
| 20 | 2.7 |
| 45 | 4.5 |
| CIMETIDINE | 34.0 |

The ability of the test compounds to inhibit histamine induced gastric secretion of acid, has been investigated after intravenous or intraduodenal administration in stomach perfused rats, according to Gosh and Schild (Br. J. Pharmacol. Chemoter. *13,* 54, 1958).

The preparation of the animals in general anaesthesia (urethane, 1 g. $kg^{-1}$i.p.) and constant temperature, is achieved by inserting and tying in place polyethyl tubes (PE 50) in the oesophagus and in the pyloricantral region. After the stomach is washed to remove residual of foods, continuous perfusion of the stomach was started with saline, 0.5 ml $min^{-1}$ (37°C), primed by a Jobling peristaltic pump. After 30 min of perfusion adaptation, the stomach perfusate is collected in 30 min samples, and titrated for acid content, expressed as µEq of NaOH 1 N. As control acid output becomes constant intravenous perfusion of histamine (1 $mg.kg^{-1}$ $hr^{-1}$) is started and maintained throughout the experimental period. After the acid secretion has reached the steadily higher level, increasing doses of the test compounds are injected intravenously in order to obtain dose response functions. $ED_{50}$ were then calculated by standard procedure.

The compounds tested by the aforementioned procedure showed a very potent antisecretory activity when administered intravenously at or below 100 $\mu g.kg^{-1}$.

The results are reported in the following Table:

TABLE 2
"In vivo" antisecretory activity in histamine induced gastric secretion (stomach perfused rat).

| Compound | $ED_{50}mg.kg^{-1}$(i.v.)* |
|---|---|
| 1 | 0.020 |
| 2 | 0.013 |
| 3 | 0.019 |
| 4 | 0.035 |
| 5 | 0.073 |
| 6 | 0.041 |
| 7 | 0.082 |
| 11 | 0.020 |
| 13 | 0.074 |
| 17 | 0.050 |
| 20 | 0.028 |
| 45 | 0.051 |
| CIMETIDINE | 0.560 |

* The values of activity are expressed taking the compound as a base.

The acute toxicity of the particularly preferred compounds of general formula (I) was approximately determined by oral administration of a single dose to groups of 5 Swiss mice fasting for 18 hours before the experiments. The evaluation of the incidence of mortality was considered after 14 days from the administration. The results are reported in the following table, in which the values are expressed taking the compound as a base.

TABLE 3

| Compound | No. Dead/No. Treated Mice |
|---|---|
| 1 | 0/5 at 500 mg/kg |
| 2 | 0/5 at 500 mg/kg |
| 3 | 0/5 at 500 mg/kg |
| 4 | 1/5 at 500 mg/kg |
| 5 | 4/5 at 500 mg/kg |
| 6 | 0/5 at 500 mg/kg |
| 7 | 0/5 at 500 mg/kg |
| 11 | 0/5 at 500 mg/kg |
| 13 | 0/5 at 500 mg/kg |
| 17 | 0/5 at 500 mg/kg |
| 20 | 0/5 at 500 mg/kg |
| 45 | 0/5 at 500 mg/kg |

According to a further feature of the present invention there are provided pharmaceutical compositions comprising as active ingredient at least one compound of formula (I), as hereinbefore defined, or a physiologically compatible acid addition salt thereof in association with a pharmaceutical carrier or excipient. For pharmaceutical administration the compounds of general formula (I) and their physiologically compatible acid addition salts may be incorporated into the conventional pharmaceutical preparations in either solid or liquid form. The compositions may, for example, be presented in a form suitable for oral or parenteral administration. Preferred forms include for example, capsules, tablets, coated tablets, ampoules or vials containing lyophilized mass of the salt.

The active ingredient may be incorporated in excipients or carriers conventionally used in pharmaceutical compositions such as for example talc, gum arabic, lactose, gelatine, magnesium stearate, corn starch, aqueous or non-aqueous vehicles, polyvinylpirrolidone, mannitol.

The compositions are advantageously formulated at dosage unit, each dosage unit being adapted to supply a fixed dose of the active ingredient. Each dosage unit may conveniently contain from 10 mg to 500 mg and preferably from 20 mg to 150 mg.

The following examples illustrate some of the new final compounds (examples 4, 14, 15, 16) or of the intermediates (examples 1 to 3 and from 5 to 13) according to the present invention; these examples are not to be in any way considered limitative of the scope of the invention itself:

Example 1

Ethyl N-[3-(2-bromoacetyl)phenyl] carbamate

To a stirred suspension of ethyl N-(3-acetylphenyl) carbamate (28.5 g), in dry dioxide (13.3 g) and dry ether (245 ml) bromine (24.1 g) was added slowly during 30 minutes. After additional 2 hours, the solution was cooled and the product which crystallized was collected by filtration, washed with ether to give 38.5 g of the title compound M.p. 108—110°C.

Example 2

Ethyl N-[3-(2-guanidino-4-thiazolyl)phenyl] carbamate, hydrobromide

A mixture of ethyl N-[3-(2-bromoacetyl)phenyl] carbamate (17.17 g), amidinothiourea (7.1 g) in ethanol (45 ml) was refluxed for 4 hours and then cooled to room temperature. The precipitate was filtered off and washed with cold ethanol to give 22 g of the title compound. M.p. 240—2°C.

Following the above procedure, using the appropriate bromoacyl starting materials, the following carbamates were prepared:

Ethyl N-[4-(2-guanidino-4-thiazolyl)phenyl] carbamate, hydrobromide
M.p. 254°C (dec.)

Ethyl N-[3-(2-methylguanidino-4-thiazolyl)phenyl] carbamate, hydrobromide
M.p. 231—233°C

Example 3

2-Guanidino-4-(3-aminophenyl) thiazole

A mixture of ethyl N-[3-(2-guanidino-4-thiazolyl)phenyl] carbamate, hydrobromide (47.1 g) in 95% ethanol (210 ml) and 50% potassium hydroxide solution (210 ml) was heated under reflux for 15 minutes. After cooling the white precipitate was filtered, dissolved in water and cautiously acidified with hydrochloric acid. The solution was made alkaline with an excess of sodium hydroxide solution and the precipitate was filtered, washed with water and dried to give 20 g of the title compound. M.p. 224—4°C.

Following the above procedure, using the appropriate carbamate starting material, the following amines were prepared:

2-Guanidino-4-(4-aminophenyl) thiazole
M.p. 251—253°C.

2-(2-Methylguanidino)-4-(3-aminophenyl) thiazole
M.p. 180—182°C.

Example 4

N-Cyano-N'-[3-(2-guanidino-4-thiazolyl)phenyl] formamidine

a) A solution of 2-guanidino-4-(3-aminophenyl) thiazole (9.33 g) and ethyl N-cyanoformamidate (3.93 g) in ethanol (65 ml) was stirred at room temperature overnight. The crystallized product was collected by filtration, washed with cold ethanol and dried to give 9.2 g of the title compound. M.p. 215°C (dec.)

a') A mixture of 2-guanidino-4-(3-aminophenyl) thiazole (23.3 g), ethyl ortoformate (18.5 g) and cyanamide (4.2 g) was heated to 120°C for 15 minutes, cooled to room temperature, treated with ethanol and filtered to give 23 g of the title compound. M.p. 214—216°C (dec).

Following the above procedures, using the appropriate amine starting materials, the following N-cyano amidines were prepared:

N-Cyano-N'-[4-(2-guanidino-4-thiazolyl)phenyl] formamidine
M.p. 240°C (dec.)

N-Cyano-N'-[2-(2-guanidino-4-thiazolyl)phenyl] formamidine
M.p. 174°C (dec.)

N-Cyano-N'-[3-(2-methylguanidino-4-thiazolyl)phenyl] formamidine
M.p. 218—220°C

N-Cyano-N'-methyl-N'-[3-(2-guanidino-4-thiazolyl)phenyl] formamidine
M.p. 216—217°C (dec.)

N-Cyano-N'-[3-(2-guanidino-5-methyl-4-thiazolyl)phenyl] formamidine
M.p. 180°C (dec.)

N-Cyano-N'-[6-methyl-3-(2-guanidino-4-thiazolyl)phenyl] formamidine
M.p. 245—248°C

N-Cyano-N'-[6-chloro-3-(2-guanidino-4-thiazolyl)phenyl] formamidine
M.p. 265—267°C (dec.)

N-Cyano-N'-[6-methoxy-3-(2-guanidino-4-thiazolyl)phenyl] formamidine
M.p. 248—250°C (dec.)

N-Cyano-N'-[4-(2-guanidino-1,3,4-thiadiazol-5-yl)phenyl] formamidine
M.p. 248—250°C (dec.)

N-Cyano-N'-[3-(2-guanidino-1,3,4-thiadiazol-5-yl)phenyl] formamidine
M.p. 259—261°C (dec.)

N-Cyano-N'-[3-(5-guanidino-1,2,4-thiadiazol-3-yl)phenyl] formamidine
M.p. 265—268°C (dec.)

N-Cyano-N'-[4-(5-guanidino-1,2,4-thiadiazol-3-yl)phenyl] formamidine
M.p. 253—255°C (dec.)

N-Cyano-N'-[3-(2-guanidino-4-oxazolyl)phenyl] formamidine
M.p. 225—226°C

N-Cyano-N'-[4-(2-guanidino-4-oxazolyl)phenyl] formamidine
M.p. 222—223°C

N-Cyano-N'-[4-(5-guanidino-1,2,4-oxadiazol-3-yl)phenyl] formamidine
M.p. > 270°C

N-Cyano-N'-[3-(5-guanidino-1,2,4-oxadiazol-3-yl)phenyl] formamidine
M.p. 246—248°C

Example 5

5-Guanidino-3-(3-nitrophenyl)-1,2,4-oxadiazole

To a solution of sodium (1.8 g) in ethanol (100 ml) was added in portions guanidine hydrochloride (7.3 g). After 30 minutes stirring 3-(3-nitrophenyl)-5-trichloromethyl-1,2,4-oxadiozole (22.5 g) was added. The solid which separated overnight was filtered and dried to give 15.2 g of the title compound. M.p. 290—291°C.

Example 6

2-Guanidino-4-(4-nitrophenyl) oxazole, hydrochloride

A solution of 4-nitrophenacyl acetate (90 g) and 1-cyanoguanidine (33.9 g) in dioxane (200 ml) and 5N hydrochloric acid (20 ml) was stirred for 2 days at room temperature. A first crop of the product was filtered; the solution, after the addition of further 5N hydrochloric acid (40 ml) was stirred for 3 days and furnished a second crop of the product. The overall yield of the title compound was 46 g. M.p. >285°C.

Example 7

2-Guanidino-4-(2-nitrophenyl) thiazole

A solution of 2-nitro-phenacyl bromide (16.5 g) and amidinothiourea (7.98 g) in dimethylformamide (70 ml) was stirred at room temperature for 16 hours, then poured into water (350 ml). The solution was made alkaline with 10% sodium hydroxide and the solid which separated after cooling was filtered, washed with water and dried to give 16.9 g of the title compound. M.p. 176—178°C.

Following the above procedure, using the appropriate phenacyl bromide as starting material, the following nitro-phenyl-thiazole derivatives were prepared:

2-Guanidino-4-(4-methyl-3-nitro-phenyl) thiazole
M.p. 225—227°C

2-Guanidino-4-(4-chloro-3-nitro-phenyl) thiazole
M.p. 248—250°C

2-Guanidino-4-(4-methoxy-3-nitro-phenyl) thiazole
M.p. 236—238°C

Example 8

2-Guanidino-4-(2-aminophenyl) thiazole

To a hot solution (60°C) of 2-guanidino-4-(2-nitrophenyl) thiazole (16.9 g) in methanol (120 ml) a solution of sodium sulfide octahydrate (57 g) in methanol (400 ml) and water (120 ml) was added dropwise over 30 minutes. After an additional 2 hours heating at 60°C, the solution was filtered with charcoal and evaporated to dryness. The residual solid was suspended in water, filtered and dried to give 12.4 g of the title compound. M.p. 198—200°C.

Following the above procedure, using the appropriate nitrophenyl derivative, the following aminophenyl derivatives were prepared:

2-Guanidino-4-(3-amino-4-methyl-phenyl) thiazole
M.p. 251—252°C.

2-Guanidino-4-(3-amino-4-chloro-phenyl) thiazole
M.p. 245—248°C.

2-Guanidino-4-(3-amino-4-methoxy-phenyl) thiazole
M.p. 157—159°C.

2-Guanidino-5-(4-aminophenyl)-1,3,4-thiadiazole
M.p. 274—275°C.

2-Guanidino-5-(3-aminophenyl)-1,3,4-thiadiazole
M.p. 244—247°C.

5-Guanidino-3-(4-aminophenyl)-1,2,4-thiadiazole
M.p. 282—284°C (as hydrochloride).

2-Guanidino-4-(4-aminophenyl) oxazole
M.p. 212—213°C.

5-Guanidino-3-(4-aminophenyl)-1,2,4-oxadiazole
M.p. 245°C (dec.)

5-Guanidino-3-(3-aminophenyl)-1,2,4-oxadiazole
M.p. 260°C (dec.)

Example 9

2-Guanidino-5-(4-nitrophenyl)-1,3,4-thiadiazole

To a 50% sodium hydride dispersion in oil (6.5 g) in dimethylformamide (200 ml) guanidine nitrate (24.13 g) was added in small portions over 30 minutes. When evolution of hydrogen has ceased, a solution of 2-chloro-5-(4-nitrophenyl)-1,3,4-thiadiazole (15.8 g) in dimethylformamide (300 ml) was dropped into the mixture, that was then stirred at room temperature for 2 hours and at 90°C for 30 minutes. After cooling and

dilution with water, the precipitate was filtered and dissolved in ethanol. Hydrogen chloride was added to this solution and the hydrochloride which separated was filtered, dissolved in water and the solution was made alkaline with 10% sodium hydroxide. The free base was filtered, washed with water and dried to give 9.5 g of the title compound. M.p. 295—297°C.

Following the above procedure, using the appropriate thiadiazole derivative as starting material, the following compounds were prepared:

2-Guanidino-5-(3-nitrophenyl)-1,3,4-thiadiazole
M.p. 296—298°C.

5-Guanidino-3-(4-nitrophenyl)-1,2,4-thiadiazole
M.p. 272—275°C.

Example 10

2-Chloro-5-(3-nitrophenyl)-1,3,4-thiadiazole

To 25% hydrochloric acid (120 ml) cooled at −10°C and containing a small amount of Cu powder was added a mixture of 2-amino-5-(3-nitrophenyl)-1,3,4-thiadiazole (14.8 g) and sodium nitrite (20.67 g) over a period of 20 minutes.

The solution was stirred at −5°C for 1 hour and at room temperature for 90 minutes then was neutralized with 10% sodium hydroxide. After addition of sodium metabisulfite solution the mixture was heated at 60°C for 10 minutes, filtered and the solid was extracted with chloroform. The organic solution was evaporated to dryness to give 12 g of the title compound. M.p. 162—165°C.

Example 11

5-Chloro-3-(4-nitrophenyl)-1,2,4-thiadiazole

To a stirred mixture of P.-nitrobenzamidine hydrochloride (101.4 g) and perchloromethyl mercaptan (70 g) in dichloromethane (760 ml) was added, keeping the temperature under −5°C, a solution of sodium hydroxide (75.6 g) in water (100 ml). After additional 2 hours at −5°C, the suspension was filtered and the organic layer was separated, evaporated to dryness and the residual solid was recrystallized from 95% ethanol to give 45 g of the title compound. M.p. 143—145°C.

Example 12

3-Methylamino-phenacyl bromide, hydrobromide

a) 3-Acetylphenyliminotriphenyl phosphorane

To a stirred suspension of triphenylphosphine dibromide (211 g) in carbon tetrachloride (1700 ml) a mixture of 3-aminoacetephenone (67.6 g) and triethylamine (101.2 g) in carbon tetrachloride (100 ml) was added. After 20 minutes refluxing, the mixture was filtered and evaporated to dryness. The residual solid was treated with isopropanol, filtered and dried to give 140.2 g of the title compound. M.p. 135—136°C.

b) [N-Methyl-N-(3-acetylphenyl)amino] triphenyl phosphonium iodide

A solution of 3-acetylphenyliminotriphenylphosphorane (140.2 g) and methyl oxide (56.9 g) in dry benzene (525 ml) was refluxed for 8 hours under an atmosphere of nitrogen. The mixture was cooled and the solid was filtered, washed with benzene and dried to give 120 g of the title compound. M.p. 178—180°C.

c) 3-Methylamino-acetophenone

[N-Methyl-N-(3-acetylphenyl)amino] triphenyl phosphonium iodide (120 g) in 2N sodium hydroxide (2250 ml) was heated under reflux for 1 hour. The mixture was cooled, acidified with hydrochloric acid, extracted with dichloromethane. The aqueous solution was made strongly alkaline with sodium hydroxide solution and the amine which separated was extracted with diethyl ether. After removal of the solvent the residue was distilled to give 25 g of the title compound. M.p. 102—103°C (0,05 mmHg).

d) 3-Methylamino-phenacyl bromide, hydrobromide

Bromide (26.8 g) in glacial acetic acid (20 ml) was added to a solution of 3-methylamino-acetophenone (25 g) and methanesulphonic acid (16.1 g) in glacial acetic acid (200 ml). After 12 hours stirring with ultraviolet light irradiation, the mixture was treated with 30 ml of 30% anhydrous hydrogen bromide in acetic acid then diluted with about 5 vol. of ether. The solid was filtered, washed with ether and recrystallized from absolute ethanol to give 20 g of the title compound. M.p. 174—176°C (dec.).

Example 13

2-Guanidino-4-(3-methylamino-phenyl) thiazole

A mixture of 3-methylamino phenacyl bromide hydrobromide (20 g), amidinothiourea (7.65 g) in ethanol (100 ml) was refluxed for 4 hours, then cooled to room temperature. The dihydrobromide which separated was filtered, dissolved in water and the solution was made alkaline with 10% sodium hydroxide. The free base was collected and dried to give 11.5 g of the title compound. M.p. 198—200°C.

Example 14

N-Methyl-N'-[3-(2-guanidino-4-thiazolyl)phenyl] formamidine (Compound 1)

N-Cyano-N'-[3-(2-guanidino-4-thiazolyl)phenyl] formamidine (4 g) was added in one portion to 35% methylamine in water (40 ml). After a few minutes from the addition, the product separated out of the solution. The solid was filtered, washed with water and dried to give 2.66 g of the title compound.

Maleate salt (ethanol) M.p. 182°C (dec.).
Analysis: $C_{20}H_{22}N_6O_8S$
Found % C 46.98 H 4.32 N 16.41
Calc. % C 47.43 H 4.38 N 16.59

The following compounds were prepared in a similar manner, starting from the above described N-cyano formamidine derivative.

N-Ethyl-N'-[3-(2-guanidino-4-thiazolyl)phenyl] formamidine (Compound 2)
Maleate salt (ethanol) M.p. 175—177°C (dec.)
Analysis: $C_{21}H_{24}N_6O_8S$
Found % C 47.95 H 4.70 N 15.98
Calc. % C 48.46 H 4.65 N 16.14

N-Propyl-N'-[3-(2-guanidino-4-thiazolyl)phenyl] formamidine (Compound 3)
Maleate salt (ethanol) M.p. 180°C (dec)
Analysis: $C_{22}H_{26}N_6O_8S$
Found % C 49.43 H 4.88 N 15.59
Calc. % C 49.43 H 4.90 N 15.72

N-Isopropyl-N'-[3-(2-guanidino-4-thiazolyl)phenyl] formamidine (Compound 4)
Citrate salt (ethanol) M.p. 135°C (dec.)
Analysis: $C_{46}H_{60}N_{12}O_{21}S_2$
Found % C 46.96 H 5.18 N 14.32
Calc. % C 46.78 H 5.12 N 14.23

N-Butyl-N'-[3-(2-guanidino-4-thiazolyl)phenyl] formamidine (Compound 5)
Maleate salt (ethanol) M.p. 196°C (dec.)
Analysis: $C_{23}H_{28}N_6O_8S$
Found % C 49.86 H 5.03 N 15.21
Calc. % C 50.36 H 5.14 N 15.32

N-sec-Butyl-N'-[3-(2-guanidino-4-thiazolyl)phenyl] formamidine (Compound 6)
Maleate salt (ethanol) M.p. 190°C (dec.)
Analysis: $C_{23}H_{28}N_6O_8S$
Found % C 49.81 H 5.18 N 15.01
Calc. % C 50.36 H 5.14 N 15.32

N-Isobutyl-N'-[3-(2-guanidino-4-thiazolyl)phenyl] formamidine (Compound 7)
Maleate salt (ethanol) M.p. 208°C (dec.)
Analysis: $C_{23}H_{28}N_6O_8S$
Found % C 50.16 H 5.16 N 15.33
Calc. % C 50.36 H 5.14 N 15.32

N-Neopentyl-N'-[3-(2-guanidino-4-thiazolyl)phenyl] formamidine (Compound 8)
Maleate salt (ethanol) M.p. 187—190°C (dec.)
Analysis: $C_{24}H_{30}N_6O_8S$
Found % C 50.73 H 5.33 N 14.81
Calc. % C 51.24 H 5.37 N 14.94

N-Hexyl-N'-[3-(2-guanidino-4-thiazolyl)phenyl] formamidine (Compound 9)
Maleate salt (ethanol) M.p. 196°C (dec.)
Analysis: $C_{25}H_{32}N_6O_8S$
Found % C 51.71 H 5.65 N 14.70
Calc. % C 52.07 H 5.59 N 14.57

N-Octyl-N'-[3-(2-guanidino-4-thiazolyl)phenyl] formamidine (Compound 10)
Maleate salt (ethanol) M.p. 177—180°C (dec.)
Analysis: $C_{27}H_{36}N_6O_8S$
Found % C 53.15 H 6.00 N 13.86
Calc. % C 53.63 H 6.00 N 13.89

N-Allyl-N'-[3-(2-guanidino-4-thiazolyl)phenyl] formamidine (Compound 11)
Maleate salt (ethanol) M.p. 168—170°C (dec.)
Analysis: $C_{22}H_{24}N_6O_8S$
Found % C 49.19 H 4.47 N 15.50
Calc. % C 49.62 H 4.54 N 15.78

N-Dimethylallyl-N'-[3-(2-guanidino-4-thiazolyl)phenyl] formamidine (Compound 12)
Maleate salt (ethanol) M.p. 163°C (dec.)
Analysis: $C_{24}H_{28}N_6O_8S$
Found % C 51.12 H 4.98 N 14.64
Calc. % C 51.42 H 5.03 N 14.99

N-Propargyl-N'-[3-(2-guanidino-4-thiazolyl)phenyl] formamidine (Compound 13)
Maleate salt (ethanol) M.p. 170°C (dec.)
Analysis: $C_{22}H_{22}N_6O_8S$
Found % C 49.06 H 4.12 N 15.89
Calc. % C 49.81 H 4.18 N 15.84

N-Cyclopropylmethyl-N'-[3-(2-guanidino-4-thiazolyl)phenyl] formamidine (Compound 14)
Maleate salt (ethanol) M.p. 183—185°C (dec.)
Analysis: $C_{23}H_{26}N_6O_8S$
Found % C 49.82 H 4.71 N 15.28
Calc. % C 50.54 H 4.79 N 15.38

N-Cyclohexyl-N'-[3-(2-guanidino-4-thiazolyl)phenyl] formamidine (Compound 15)
Maleate salt (ethanol) M.p. 190°C (dec.)
Analysis: $C_{25}H_{30}N_6O_8S$
Found % C 52.00 H 5.34 N 14.81
Calc. % C 52.26 H 5.26 N 14.63

N-Norbornyl-N'-[3-(2-guanidino-4-thiazolyl)phenyl] formamidine (Compound 16)
Maleate salt (ethanol) M.p. 196°C (dec.)
Analysis: $C_{26}H_{30}N_6O_8S$
Found % C 53.15 H 5.22 N 14.21
Calc. % C 53.23 H 5.15 N 14.33

N-(2-Methoxyethyl-N'-[3-(2-guanidino-4-thiazolyl)phenyl] formamidine (Compound 17)
Maleate salt (ethanol) M.p. 182—184°C (dec.)
Analysis: $C_{22}H_{26}N_6O_9S$
Found % C 47.67 H 4.71 N 15.15
Calc. % C 48.00 H 4.76 N 15.26

N-(2-Methylthioethyl)-N'-[3-(2-guanidino-4-thiazolyl)phenyl] formamidine (Compound 18)
Maleate salt (ethanol) M.p. 180—183°C (dec.)
Analysis: $C_{22}H_{26}N_6O_8S_2$
Found % C 46.13 H 4.57 N 14.71
Calc. % C 46.63 H 4.62 N 14.83

N-(2-cyanoethyl)-N'-[3-(2-guanidino-4-thiazolyl)phenyl] formamidine (Compound 19)
Maleate salt (ethanol) M.p. 160—164°C (dec.)
Analysis: $C_{22}H_{23}N_7O_8S$
Found % C 48.03 H 4.16 N 18.15
Calc. % C 48.44 H 4.25 N 17.97

N-(3-Hydroxypropyl)-N'-[3-(2-guanidino-4-thiazolyl)phenyl] formamidine (Compound 20)
Maleate salt (ethanol) M.p. 184°C (dec.)
Analysis: $C_{22}H_{26}N_6O_9S$
Found % C 47.08 H 4.57 N 16.95
Calc. % C 46.98 H 4.66 N 17.06

Following the above procedure, using the appropriate N-cyano formamidine derivative as starting material, the following amidines were prepared:

N-Methyl-N'-[4-(2-guanidino-4-thiazolyl)phenyl] formamidine (Compound 21)
Hydrochloride salt (ethanol) M.p. 264°C (dec.)
Analysis: $C_{12}H_{16}Cl_2N_6S$
Found % C 41.25 H 4.55 N 24.31
Calc. % C 41.50 H 4.64 N 24.20

N-Ethyl-N'-[4-(2-guanidino-4-thiazolyl)phenyl] formamidine (Compound 22)
Fumarate salt (ethanol) M.p. 136°C (dec.)
Analysis: $C_{21}N_{24}N_6O_8S$
Found % C 48.37 H 4.85 N 16.16
Calc. % C 48.46 H 4.65 N 16.14

N-Isopropyl-N'-[4-(2-guanidino-4-thiazolyl)phenyl] formamidine (Compound 23)
M.p. 249—250°C (dec.)
Analysis: $C_{14}H_{18}N_6S$
Found % C 56.07 H 6.04 N 27.32
Calc. % C 55.61 H 6.00 N 27.79

N-Ethyl-N'-[2-(2-guanidino-4-thiazolyl)phenyl] formamidine (Compound 24)
Tartrate salt (ethanol) M.p. 110°C (dec.)
Analysis: $C_{38}H_{50}N_{12}O_{18}S_2$
Found % C 44.03 H 5.14 N 16.67
Calc. % C 44.44 H 4.91 N 16.36

N-Propyl-N'-[2-(2-guanidino-4-thiazolyl)phenyl] formamidine (Compound 25)
Tartrate salt (ethanol) M.p. 135°C (dec.)
Analysis: $C_{40}H_{54}N_{12}O_{18}S_2$
Found % C 45.53 H 5.20 N 15.70
Calc. % C 45.53 H 5.16 N 15.93

N-Allyl-N'-[2-(2-guanidino-4-thiazolyl)phenyl] formamidine (Compound 26)
Tartrate salt (ethanol) M.p. 120°C (dec.)
Analysis: $C_{40}H_{50}N_{12}O_{18}S_2$
Found % C 45.51 H 4.68 N 16.12
Calc. % C 45.71 H 4.79 N 15.99

N-Ethyl-N'-[3-(2-methylguanidino-4-thiazolyl)phenyl] formamidine (Compound 27)
Maleate salt (ethanol) M.p. 183°C (dec.)
Analysis: $C_{22}H_{26}N_6O_8S$
Found % C 49.12 H 4.94 N 15.59
Calc. % C 49.43 H 4.90 N 15.72

N-Propyl-N'-[3-(2-methylguanidino-4-thiazolyl)phenyl] formamidine (Compound 28)
Maleate salt (ethanol) M.p. 172°C (dec.)
Analysis: $C_{23}H_{28}N_6O_8S$
Found % C 50.51 H 5.11 N 15.45
Calc. % C 50.36 H 5.14 N 15.32

N-Isopropyl-N'-[3-(2-methylguanidino-4-thiazolyl)phenyl] formamidine (Compound 29)
Maleate salt (ethanol) M.p. 95°C (dec.)
Analysis: $C_{23}H_{28}N_6O_8S$
Found % C 50.25 H 5.16 N 15.10
Calc. % C 50.36 H 5.14 N 15.32

N,N-Dimethyl-N'-[3-(2-guanidino-4-thiazolyl)phenyl] formamidine (Compound 30)
Maleate salt (ethanol) M.p. 216°C (dec.)
Analysis: $C_{21}H_{24}N_6O_8S$
Found % C 48.61 H 4.70 N 16.26
Calc. % C 48.46 H 4.65 N 16.15

N,N-Diethyl-N'-[3-(2-guanidino-4-thiazolyl)phenyl] formamidine (Compound 31)
Maleate salt (ethanol) M.p. 190—193°C (dec.)
Analysis: $C_{23}H_{28}N_6O_8S$
Found % C 49.99 H 5.06 N 15.32
Calc. % C 50.36 H 5.14 N 15.32

N-[3-(2-guanidino-4-thiazolyl)phenyl]-1-pyrrolidinylmethanimine (Compound 32)
Maleate salt (ethanol) M.p. 197—199°C (dec.)
Analysis: $C_{23}H_{26}N_6O_8S$
Found % C 50.87 H 4.86 N 15.08
Calc. % C 50.54 H 4.97 N 15.38

N-[3-(2-guanidino-4-thiazolyl)phenyl]-4-morpholinylmethanimine (Compound 33)
Maleate salt (ethanol) M.p. 208—210°C (dec.)
Analysis: $C_{23}H_{26}N_6O_9S$
Found % C 48.88 H 4.70 N 15.05
Calc. % C 49.10 H 4.66 N 14.94

N-Propyl-N'-methyl-N'-[3-(2-guanidino-4-thiazolyl)phenyl] formamidine (Compound 34)
Maleate salt (ethanol) M.p. 105—106°C (dec.)
Analysis: $C_{23}H_{28}N_6O_8S$
Found % C 50.21 H 4.99 N 15.45
Calc. % C 50.36 H 5.14 N 15.32

N-Ethyl-N'-[3-(2-guanidino-5-methyl-4-thiazolyl)phenyl] formamidine (Compound 37)
Maleate salt (ethanol) M.p. 155°C (dec.)
Analysis: $C_{22}H_{26}N_6O_8S$
Found % C 49.19 H 4.82 N 15.43
Calc. % C 49.43 H 4.90 N 15.72

N-Propyl-N'-[3-(2-guanidino-5-methyl-4-thiazolyl)phenyl] formamidine (Compound 38)
Maleate salt (ethanol) M.p. 160°C (dec.)
Analysis: $C_{23}H_{28}N_6O_8S$
Found % C 49.86 H 4.93 N 15.52
Calc. % C 50.36 H 5.14 N 15.32

N-Propyl-N'-[6-methyl-3-(2-guanidino-4-thiazolyl)phenyl] formamidine (Compound 39)
Maleate salt (ethanol) M.p. 204—207°C (dec.)
Analysis: $C_{23}H_{28}N_6O_8S$
Found % C 50.00 H 5.25 N 15.45
Calc. % C 50.36 H 5.15 N 15.32

N-Propyl-N'-[6-chloro-3-(2-guanidino-4-thiazolyl)phenyl] formamidine (Compound 40)
Maleate salt (ethanol) M.p. 192—195°C (dec.)
Analysis: $C_{22}H_{25}ClN_6O_8S$
Found % C 46.74 H 4.51 N 14.54
Calc. % C 46.44 H 4.43 N 14.77

N-Propyl-N'-[6-methoxy-3-(2-guanidino-4-thiazolyl)phenyl] formamidine (Compound 41)
Maleate salt (ethanol) M.p. 201—204°C (dec.)
Analysis: $C_{23}H_{28}N_6O_9S$
Found % C 49.19 H 5.07 N 14.74
Calc. % C 48.93 H 4.99 N 14.89

N-Ethyl-N'-[4-(2-guanidino-1,3,4-thiadiazolyl-5-yl)phenyl] formamidine (Compound 42)
Maleate salt (ethanol) M.p. 182—183°C (dec.)
Analysis: $C_{20}H_{23}N_7O_8S$
Found % C 46.18 H 4.49 N 18.70
Calc. % C 46.06 H 4.45 N 18.80

N-Isopropyl-N'-[4-(2-guanidino-1,3,4-thiadiazol-5-yl)phenyl] formamidine (Compound 43)
Maleate salt (ethanol) M.p. 178°C (dec.)
Analysis: $C_{21}H_{25}N_7O_8S$
Found % C 46.95 H 4.77 N 18.42
Calc. % C 47.09 H 4.71 N 18.31

N-Isopropyl-N'-[3-(2-guanidino-1,3,4-thiadiazol-5-yl)phenyl] formamidine (Compound 44)
Maleate salt (ethanol) M.p. 166°C (dec.)
Analysis: $C_{21}H_{25}N_7O_8S$
Found % C 46.75 H 4.68 N 18.35
Calc. % C 47.09 H 4.71 N 18.31

N-Ethyl-N'-[3-(5-guanidino-1,2,4-thiadiazol-3-yl)phenyl] formamidine (Compound 45)
Maleate salt (ethanol) M.p. 173—175°C (dec.)
Analysis: $C_{20}H_{23}N_7O_8S$
Found % C 45.92 H 4.50 N 18.69
Calc. % C 46.06 H 4.45 N 18.80

N-Isopropyl-N'-[3-(5-guanidino-1,2,4-thiadiazol-3-yl)phenyl] formamidine (Compound 46)
Maleate salt (ethanol) M.p. 157°C (dec.)
Analysis: $C_{21}H_{25}N_7O_8S$
Found % C 46.73 H 4.71 N 18.21
Calc. % C 47.09 H 4.71 N 18.31

N-Ethyl-N'-[4-(5-guanidino-1,2,4-thiadiazol-3-yl)phenyl] formamidine (Compound 47)
Maleate salt (acetone) M.p. 184°C (dec.)
Analysis: $C_{20}H_{23}N_7O_8S$
Found % C 45.70 H 4.39 N 18.68
Calc. % C 46.06 H 4.45 N 18.80

N-Propyl-N'-[4-(5-guanidino-1,2,4-thiadiazol-3-yl)phenyl] formamidine (Compound 48)
Maleate salt (acetone) M.p. 187°C (dec.)
Analysis: $C_{21}H_{25}N_7O_8S$
Found % C 46.86 H 4.64 N 18.42
Calc. % C 47.09 H 4.71 N 18.31

N-Isopropyl-N'-[4-(5-guanidino-1,2,4-thiadiazol-3-yl)phenyl] formamidine (Compound 49)
Maleate salt (acetone) M.p. 177°C (dec.)
Analysis: $C_{21}H_{25}N_7O_8S$
Found % C 46.95 H 4.66 N 18.27
Calc. % C 47.09 H 4.71 N 18.31

N-Methyl-N'-[3-(2-guanidino-4-oxazolyl)phenyl] formamidine (Compound 50)
Hydrochloride salt (ethanol) M.p. 252—253°C (dec.)
Analysis: $C_{12}H_{16}Cl_2N_6O$
Found % C 43.26 H 4.83 N 25.37
Calc. % C 43.51 H 4.87 N 25.37

N-Ethyl-N'-[3-(2-guanidino-4-oxazolyl)phenyl] formamidine (Compound 51)
Hydrochloride salt (ethanol) M.p. 254—255°C (dec.)
Analysis: $C_{13}H_{18}Cl_2N_6O$
Found % C 44.82 H 5.15 N 24.12
Calc. % C 45.22 H 5.22 N 24.34

N-Hexyl-N'-[3-(2-guanidino-4-oxazolyl)phenyl] formamidine (Compound 52)
Hydrochloride salt (ethanol) M.p. 237—238°C (dec.)
Analysis: $C_{17}H_{26}Cl_2N_6O$
Found % C 50.17 H 6.57 N 21.09
Calc. % C 50.87 H 6.53 N 20.94

N-Methyl-N'-[4-(2-guanidino-4-oxazolyl)phenyl] formamidine (Compound 53)
Hydrochloride salt (ethanol) M.p. 239—240°C (dec.)
Analysis: $C_{12}H_{16}Cl_2N_6O$
Found % C 43.25 H 4.87 N 24.91
Calc. % C 43.51 H 4.87 N 25.37

N-Propyl-N'-[4-(2-guanidino-4-oxazolyl)phenyl] formamidine (Compound 54)
Hydrochloride salt (ethanol) M.p. 232—233°C (dec.)
Analysis: $C_{14}H_{20}Cl_2N_6O$
Found % C 47.02 H 5.58 N 23.46
Calc. % C 46.80 H 5.61 N 23.39

N-Butyl-N'-[4-(2-guanidino-4-oxazolyl)phenyl] formamidine (Compound 55)
Hydrochloride salt (ethanol) M.p. 233—235°C (dec.)
Analysis: $C_{15}H_{22}Cl_2N_6O$
Found % C 48.41 H 5.99 N 22.14
Calc. % C 48.26 H 5.94 N 22.52

N-Allyl-N'-[4-(2-guanidino-4-oxazolyl)phenyl] formamidine (Compound 56)
Fumarate salt (ethanol) M.p. 150—152°C (dec.)
Analysis: $C_{18}H_{20}N_6O_5$
Found % C 53.87 H 5.06 N 21.16
Calc. % C 53.99 H 5.04 N 20.99

N-Isopropyl-N'-[4-(5-guanidino-1,2,4-oxadiazol-3-yl)phenyl] formamidine (Compound 57)
Maleate salt (ethanol) M.p. 189°C (dec.)
Analysis: $C_{21}H_{25}N_7O_9$
Found % C 48.10 H 4.86 N 18.61
Calc. % C 48.55 H 4.85 N 18.87

N-Methyl-N'-[4-(5-guanidino-1,2,4-oxadiazol-3-yl)phenyl] formamidine (Compound 58)
Fumarate salt (ethanol) M.p. 186—187°C (dec.)
Analysis: $C_{15}H_{17}N_7O_5$
Found % C 47.85 H 4.57 N 25.91
Calc. % C 48.00 H 4.57 N 26.12

N-Isopropyl-N'-[3-(5-guanidino-1,2,4-oxadiazol-3-yl)phenyl] formamidine (Compound 59)
Maleate salt (ethanol) M.p. 167°C (dec.)
Analysis: $C_{21}H_{25}N_7O_9$
Found % C 48.96 H 4.85 N 19.05
Calc. % C 48.55 H 4.85 N 18:87

N-Methyl-N'-[3-(5-guanidino-1,2,4-oxadiazol-3-yl)phenyl] formamidine (Compound 60)
Nitrate salt (methanol) M.p. 215—216°C (dec.)
Analysis: $C_{11}H_{15}N_9O_7$
Found % C 33.97 H 3.88 N 32.58
Calc. % C 34.28 H 3.92 N 32.72

N-Allyl-N'-[3-(5-guanidino-1,2,4-oxadiazol-3-yl)phenyl] formamidine (Compound 61)
Maleate salt (ethanol) M.p. 160°C (dec.)
Analysis: $C_{21}H_{23}N_7O_9$
Found % C 48.21 H 4.48 N 18.80
Calc. % C 48.74 H 4.47 N 18.95

N-sec-Butyl-N'-[3-(5-guanidino-1,2,4-oxadiazol-3-yl)phenyl] formamidine (Compound 62)
Maleate salt (ethanol) M.p. 170°C (dec.)
Analysis: $C_{22}H_{27}N_7O_9$
Found % C 49.24 H 5.18 N 18.52
Calc. % C 49.53 H 5.10 N 18.38

Example 15

N,N-Dimethyl-N'-[3-(2-guanidino-4-thiazolyl)phenyl] formamidine (Compound 30)

A solution of 2-guanidino-4-(3-aminophenyl) thiazole (2.5 g) in N,N-dimethylformamide diethylacetal (7.5 g) was stirred at room temperature for 2 days. Addition of diethyl ether afforded precipitation of a solid which was filtered and dried to give 2.4 g of the title compound.

Maleate salt (ethanol) M.p. 214—215°C (dec.)

The following formamidine was prepared in a similar manner starting from N,N-diethylformamide diethylacetal:

N,N-Diethyl-N'-[3-(2-guanidino-4-thiazolyl)phenyl] formamidine (Compound 31)
Maleate salt M.p. 190—192°C (dec.)

Example 16

N-(2-Pyridyl)-N'-[3-(2-guanidino-4-thiazolyl)phenyl] formamidine (Compound 35)

To a solution of ethyl N-(2-pyridyl) formamidate (9.65 g) in ethanol (120 ml) was added 2-guanidino-4-(3-aminophenyl) thiazole (5 g). After stirring for 90 minutes at room temperature the solid which separated

was filtered and dried to give 3 g of the title compound.

M.p. 140°C (dec.)

Analysis: $C_{16}H_{15}N_7S$

| | | | |
|---|---|---|---|
| Found % | C 57.14 | H 4.41 | N 28.86 |
| Calc. % | C 56.96 | H 4.48 | N 29.06 |

The following formamidine was prepared in a similar manner using ethyl N-phenyl-formamidate: N-Phenyl-N'-[3-(2-guanidino-4-thiazolyl)phenyl] formamidine (Compound 36)

M.p. 167—168°C (dec.)

Analysis: $C_{17}H_{16}N_6S$

| | | | |
|---|---|---|---|
| Found % | C 60.10 | H 4.74 | N 24.56 |
| Calc. % | C 60.69 | H 4.79 | N 24.98 |

The following not limitative examples of pharmaceutical compositions according to the invention are reported:

## Example 17

Tablets

| | |
|---|---|
| — active ingredient | 50 mg |
| — lactose | 217 mg |
| — corn starch | 30 mg |
| — magnesium stearate | 3 mg |

Method of preparation: the active ingredient, lactose, and corn starch were mixed and homogeneously moistened with water. After screening of the moist mass and drying in a tray driver, the mixture was again passed through a screen and magnesium stearate was added. Then the mixture was pressed into tablets weighing 300 mg each. Each tablet contains 50 mg of active ingredient.

## Example 18

Capsules

| | |
|---|---|
| — active ingredient | 50 mg |
| — corn starch | 170 mg |
| — magnesium stearate | 2 mg |

Method of preparation: the active ingredient was mixed with the auxiliary products, and the mixture was passed through a screen and mixed homogeneously in a suitable device. The resulting mixture was filled into hard gelatine capsule (222 mg per capsule); each capsule contains 50 mg of active ingredient.

## Example 19

Vials

| | |
|---|---|
| — active ingredient | 50 mg |
| — water for injection q.s. | 5 ml |

Method of preparation: the active ingredient was dissolved in the water in appropriate amounts, and then the resulting solution was introduced into 5 ml vials under sterile conditions. Each vial contains 50 mg of active ingredient.

**Claims for the Contracting States: BE CH DE FR IT LI LU NL SE**

1. Compound of general formula (I)

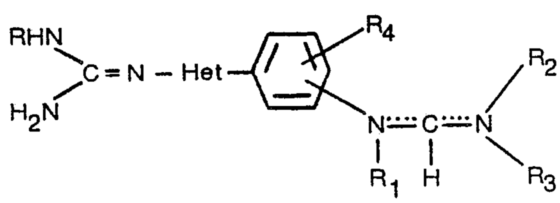

(I)

wherein R, $R_1$ and $R_2$, which may be the same or different, each represents a hydrogen atom or an alkyl group having 1 to 4 carbon atoms, $R_3$ represents a linear or branched $C_1$—$C_8$ alkyl group containing optionally heteroatoms such as oxygen sulfur or nitrogen atom, a linear or branched $C_3$—$C_5$ alkenyl group, a $C_3$—$C_4$ alkynyl group, a cyano group, a $C_3$—$C_5$ cycloalkyl or cycloaliphatic alkyl group, a norbornyl group, a phenyl group, an unsaturated six-membered heterocyclic ring; or $R_3$ and $R_2$ together with the

adjacent nitrogen atom represent a saturated five or six-membered heterocyclic ring which may contain a further heteroatom, $R_4$ represents a hydrogen atom, an alkyl or alkoxy group both having 1 to 4 carbon atoms, or halogen atom, Het represents a substituted or unsubstituted five-membered heterocyclic ring containing two or three heteroatoms, and tautomers thereof and acid addition salts of the aforesaid compounds, the dotted line denoting that a double bond between the C atom and one of the N atoms is present, and that either $R_1$ or $R_2$ are not present, respectively.

2. Physiologically compatible acid addition salts of compounds of formula (I) as claimed in claim 1.

3. Salts as claimed in claim 2 formed with hydrochloric, sulphuric, maleic or fumaric acid.

4. Compounds as claimed in any one of the preceding claims wherein the amidine radical is in the meta position of the benzene ring with respect to the Het group.

5. Compounds as claimed in any one of the preceding claims wherein R, $R_1$, and $R_4$ are hydrogen atoms, $R_2$ is absent, $R_3$ is methyl, ethyl, n-propyl, isopropyl, n-butyl, isobutyl, sec-butyl, neopentyl, n-hexyl, n-octyl, allyl, dimethylallyl, propargyl, cyclopropylmethyl, cyclohexyl, norbornyl, phenyl, pyridil, hydroxypropyl, methoxyethyl, methylthioethyl or cyanoethyl group, and Het is thiazolyl or 1,2,4-thiadiazolyl group.

6. N-Methyl-N'-[3-(2-guanidino-4-thiazolyl) phenyl] formamidine

7. N-Ethyl-N'-[3-(2-guanidino-4-thiazolyl) phenyl] formamidine

8. N-Propyl-N'-[3-(2-guanidino-4-thiazolyl) phenyl] formamidine

9. N-Isopropyl-N'-[3-(2-guanidino-4-thiazolyl) phenyl] formamidine

10. N-Butyl-N'-[3-(2-guanidino-4-thiazolyl) phenyl] formamidine

11. N-sec-Butyl-N'-[3-(2-guanidino-4-thiazolyl) phenyl] formamidine

12. N-Isobutyl-N'-[3-(2-guanidino-4-thiazolyl) phenyl] formamidine

13. N-Allyl-N'-[3-(2-guanidino-4-thiazolyl) phenyl] formamidine

14. N-Propargyl-N'-[3-(2-guanidino-4-thiazolyl) phenyl] formamidine

15. N-(2-Methoxyethyl)-N'-[3-(2-guanidino-4-thiazolyl) phenyl] formamidine

16. N-(3-Hydroxypropyl)-N'-[3-(2-guanidino-4-thiazolyl) phenyl] formamidine

17. N-Ethyl-N'-[3-(5-guanidino-1,2,4-thiadiazol-3-yl) phenyl] formamidine

18. Physiologically compatible acid addition salts of compounds as claimed in claims 6 to 17.

19. Hydrochloric, sulphuric, maleic or fumaric acid addition salts of compounds as claimed in claims 6 to 17.

20. A process for the preparation of compounds of general formula (I) as claimed in claim 1 which comprises reacting a compound of formula (II)

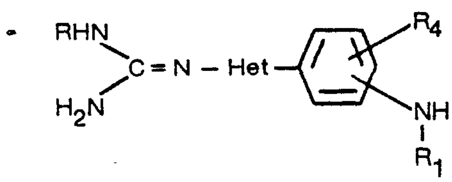

(II)

(wherein R, $R_1$, $R_4$ and Het are as defined in claim 1) with a compound of formula (III)

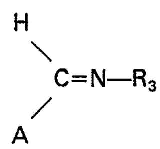

(III)

in which $R_3$ is as defined in claim 1 and A represents a methoxy or ethoxy group.

21. A process as claimed in claim 20 in which the reaction is carried out in the presence of an inert organic solvent.

22. A process as claimed in claim 21 in which the inert organic solvent comprises ethanol, dioxan or acetone.

23. A process as claimed in claims 20 to 22 in which the reaction is carried out at a temperature of from 20 to 60°C.

24. A process for the preparation of compounds of general formula (I) as claimed in claim 1 which comprises reacting a compound of formula (VI)

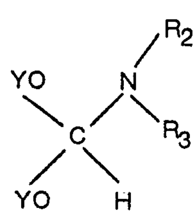

(VI)

in which $R_2$, $R_3$ are as defined in claim 1 and Y represents a methyl or ethyl group, with an amine of formula (II) as defined in claim 20.

25. A process as claimed in claim 24 in which the reaction is carried out at a temperature of from 20 to 80°C.

26. A process for the preparation of compounds of general formula (I) as claimed in claim 1 which comprises reacting a compound of formula (VIIa) or (VIIb).

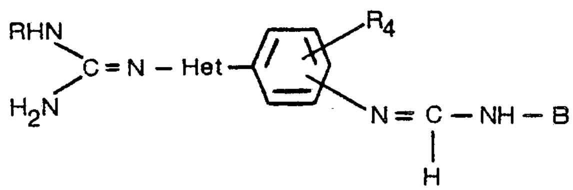

(VIIa)

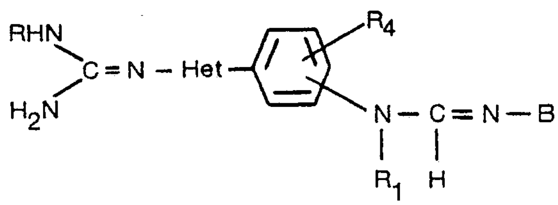

(VIIb)

in which R, $R_1$, $R_4$ and Het are as defined in claim 1 and B represents a cyano, acetyl, carbethoxy or carbamyl group, with an amine of formula (IV)

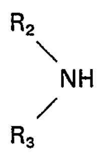

(IV)

(wherein $R_2$ and $R_3$ are as defined in claim 1).

27. A process as claimed in claim 26 in which the reaction is carried out in the presence of water or of dimethylformamide.

28. A process as claimed in claims 26 and 27 in which the reaction is effected at room temperature.

29. Pharmaceutical compositions comprising as active ingredient at least one compound of general formula (I) as defined in claim 1 or a tautomer or physiologically compatible acid addition salt thereof, in association with a pharmaceutical carrier or excipient.

30. Pharmaceutical compositions as claimed in claim 29 for use as anti-ulcers.

31. Pharmaceutical compositions as claimed in claim 29 for treatment of patients suffering from disorders of gastrointestinal tract.

32. New intermediate compounds of formula (VIIa) and (VIIb)

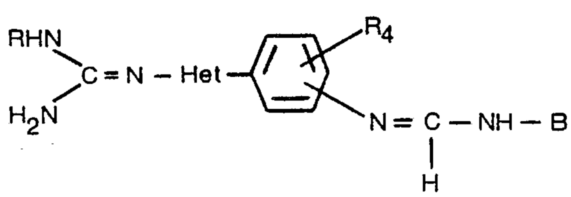

(VIIa)

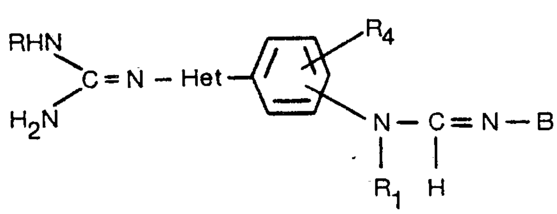

(VIIb)

wherein R, $R_1$, $R_4$ and Het are as defined in claim 1 and B is as defined in claim 26 for the performance of the process as claimed in claim 26.

**Claims for the Contracting State: AT**

1. A process for the preparation of compounds of general formula (I)

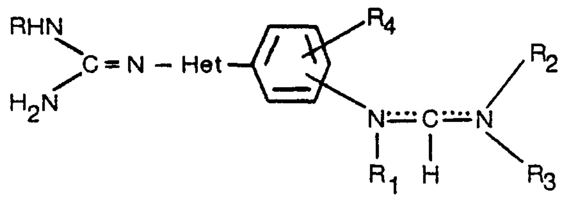

(I)

wherein R, $R_1$ and $R_2$, which may be the same or different, each represents a hydrogen atom or an alkyl group having 1 to 4 carbon atoms, $R_3$ represents a linear or branched $C_1$—$C_8$ alkyl group containing optionally heteroatoms such as oxygen sulfur or nitrogen atom, a linear or branched $C_3$—$C_5$ alkenyl group, a $C_3$—$C_4$ alkynyl group, a cyano group, a $C_3$—$C_5$ cycloalkyl or cycloaliphatic alkyl group, a norbornyl group, a phenyl group, an unsaturated six-membered heterocyclic ring; or $R_3$ and $R_2$ together with the adjacent nitrogen atom represent a saturated five or six-membered heterocyclic ring which may contain a further heteroatom, $R_4$ represents a hydrogen atom, an alkyl or alkoxy group both having 1 to 4 carbon atoms, or halogen atom, Het represents a substituted or unsubstituted five-membered heterocyclic ring containing two or three heteroatoms, and tautomers thereof and acid addition salts of the aforesaid compounds, the dotted line denoting that a double bond between the C atom and one of the N atoms is present, and that either $R_1$ or $R_2$ are not present, respectively, characterized by reacting a compound of formula (II)

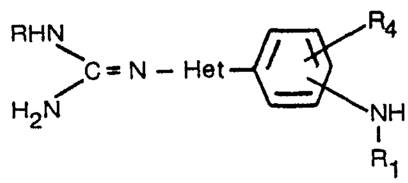

(II)

(wherein R, $R_1$, $R_4$ and Het are as defined in claim 1) with a compound of formula (III)

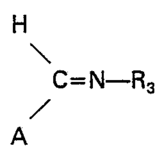

(III)

in which $R_3$ is as defined in claim 1 and A represents a methoxy or ethoxy group.

2. A process as claimed in Claim 1 in which the reaction is carried out in the presence of an inert organic solvent.

3. A process as claimed in Claim 2 in which the inert organic solvent comprises ethanol, dioxan or acetone.

4. A process as claimed in Claims 1 to 3 in which the reaction is carried out at a temperature of from 20 to 60°C.

5. A process for the preparation of compounds of general formula (I) as claimed in Claim 1 which comprises reacting a compound of formula (VI)

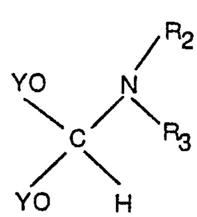

(VI)

in which $R_2$, $R_3$ are as defined in Claim 1 and Y represents a methyl or ethyl group, with an amine of formula (II) as defined in Claim 2.

6. A process as claimed in Claim 5 in which the reaction is carried out at a temperature of from 20 to 80°C.

7. A process for the preparation of compounds of general formula (I) as claimed in Claim 1 which comprises reacting a compound of formula (VII a) or (VII b)

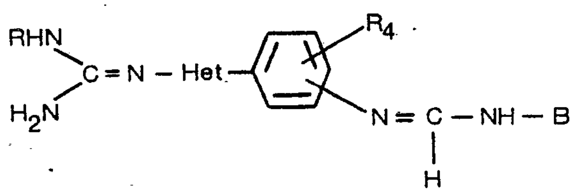

(VIIa)

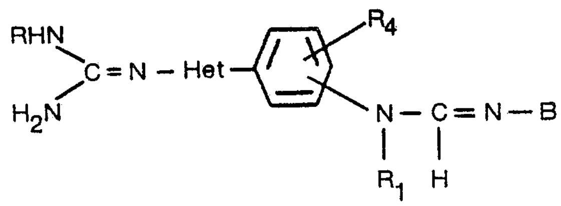

(VIIb)

in which R, $R_1$, $R_4$ and Het are as defined in claim 1 and B represents a cyano, acetyl, carbethoxy or carbamyl group, with an amine of formula (IV)

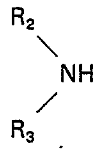

(IV)

(wherein $R_2$ and $R_3$ are as defined in claim 1).

8. A process as claimed in claim 7 in which the reaction is carried out in the presence of water or of dimethylformamide.

9. A process as claimed in Claims 7 and 8 in which the reaction is effected at room temperature.

**Patentansprüche für die Vertragsstaaten: BE CH DE FR IT LI LU NL SE**

1. Verbindung der allgemeinen Formel (I)

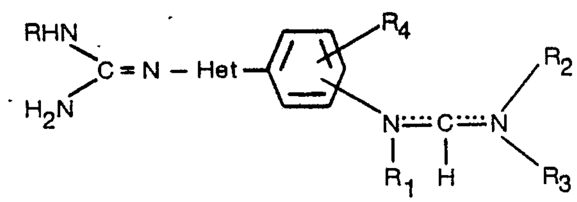

(I)

worin die untereinander gleichen oder voneinander verschiedenen Reste R, $R_1$ und $R_2$ je für Wasserstoff oder eine Alkylgruppe mit 1 bis 4 C-Atomen stehen, $R_3$ eine geradkettige oder verzweigtkettige und gegebenenfalls Heteroatome wie Sauerstoff, Schwefel oder Stickstoff enthaltende Alkylgruppe, eine geradkettige oder verzweigtkettige Alkenylgruppe mit 3 bis 5 C-Atomen, eine Alkinylgruppe mit 3 oder 4 C-Atomen, eine Cyanogruppe, eine $C_3$—$C_6$ Cycloalkylgruppe oder eine Cycloaliphatische Gruppe, eine Norbornylgruppe, eine Phenylgruppe, ein ungesättigter sechsgliedriger hetrocyclischer Ring; oder $R_3$ und $R_2$ gemeinsam mit dem angrenzenden Stickstoffatom einen fünf- oder sechsgliedrigen heterocyclischen Ring, der auch ein weiteres Heteroatom enthalten kann, bilden, $R_4$ Wasserstoff, eine Alkyl- oder Alkoxygruppe mit 1 bis 4 C-Atomen oder ein Halogenatom bedeutet, Het stellt einen substituierten oder unsubstituierten fünf- oder sechsgliedrigen hetrocyclischen Ring dar, der zwei oder drei Heteroatome enthält, und von Tautomeren und von Säureadditionssalzen hievon; die gepunktete Linie in der Verbindung besagt, daß eine Doppelbindung zwischen dem C-Atom und einem der Stickstoffatome besteht und daß entweder $R_1$ oder $R_2$ nicht worhanden ist.

2. Physiologisch verträgliche Säureadditionssalze der Verbindungen der Formel (I) nach Anspruch 1.

3. Salze nach Anspruch 2, die mit Chlorwasserstoffsäure, Schwefelsäure, Maleinsäure oder Fumarsäure gebildet wurden.

4. Verbindungen nach einem der vorhergehenden Ansprüche, dadurch gekennzeichnet, daß das Amidinradikal an dem Benzolring in Bezug auf die Het-Gruppe in meta-Stellung liegt.

5. Verbindungen nach einem der vorhergehenden Ansprüche, dadurch gekennzeichnet, daß R, $R_1$ und $R_4$ Wasserstoffatome sind, $R_2$ nicht vorhanden ist, $R_3$ Methyl, Ethyl, n-Propyl, Isopropyl, n-Butyl, Isobutyl, sec-Butyl, Neopentyl, n-Hexyl, n-Octyl, Allyl, Dimethylallyl, Propargyl, Cyclopropylmethyl, Cyclohexyl, Norbornyl, Phenyl, Pyridil, Hydroxypropyl, Methoxyethyl, Methylthioethyl oder eine Cyanoethylgruppe ist und Het eine Thiazolyl oder 1,2,4-Thiadiazolylgruppe ist.

6. N-Methyl-N'-[3-(2-guanidino-4-thiazolyl) phenyl]-formamidin.
7. N-Ethyl-N'-[3-(2-guanidino-4-thiazolyl) phenyl]-formamidin.
8. N-Propyl-N'-[3-(2-guanidino-4-thiazolyl) phenyl]-formamidin.
9. N-Isopropyl-N'-[3-(2-guanidino-4-thiazolyl) phenyl]-formamidin.
10. N-Butyl-N'-[3-(2-guanidino-4-thiazolyl) phenyl]-formamidin.
11. N-sec-Butyl-N'-[3-(2-guanidino-4-thiazolyl) phenyl]-formamidin.
12. N-Isobutyl-N'-[3-(2-guanidino-4-thiazolyl)phenyl]-formamidin.
13. N-Allyl-N'-[3-(2-guanidino-4-thiazolyl) phenyl]-formamidin.
14. N-Propargyl-N'-[3-(2-guanidino-4-thiazolyl) phenyl]-formamidin.
15. N-(2-Methoxyethyl)-N'-[3-(2-guanidino-4-thiazolyl) phenyl]-formamidin.
16. N-(3-Hydroxypropyl)-N'-[3-(2-guanidino-4-thiazolyl) phenyl]-formamidin.
17. N-Ethyl-N'-[3-(5-guanidino-1,2,4-thiadiazol-3-yl) phenyl]-formamidin.
18. Physiologisch verträgliche Säureadditionssalze der Verbindung nach den Ansprüchen 6 bis 17.
19. Chlorwasserstoff, Schwefelsäure, Maleinsäure oder Fumarsäure Additionssalze der Verbindunge nach den Ansprüchen 6 bis 17.
20. Verfahren zum Herstellung von Verbindungen der allgemeinen Formel (I) der in Anspruch 1 beanspruchten Art, bei welchem eine Verbindung der Allgemeinen Formel (II)

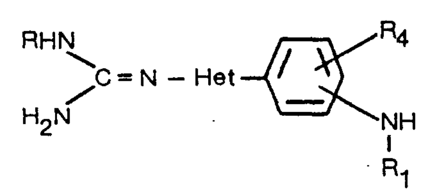
(II)

worin R, $R_1$, $R_4$ und Het die in Anspruch 1 angegebenen Bedeutungen besitzen, mit einer Verbindung der allgemeinen Formel (III)

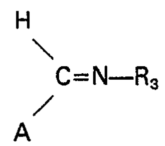
(III)

in welcher $R_3$ die in Anspruch 1 angegebene Bedeutung besitzt und A eine Methoxy- oder Ethoxygruppe darstellt, umgesetzt wird.
21. Verfahren nach Anspruch 20, dadurch gekennzeichnet, daß die Umsetzung in Anwesenheit eines inerten organischen Lösungsmittels vorgenommen wird.
22. Ein Verfahren nach Anspruch 21, dadurch gekennzeichnet, daß das inerte organische Lösungsmittel Ethanol, Dioxan oder Aceton ist.
23. Ein Verfahren nach den Ansprüchen 20 bis 22, dadurch gekennzeichnet, daß die Umsetzung bei einer Temperatur von 20 bis 60°C vorgenommen wird.
24. Verfahren zum Herstellen von Verbindungen der allgemeinen Formel (I) der in Anspruch 1 beanspruchten Art, bei welchem eine Verbindung der allgemeinen Formel (VI)

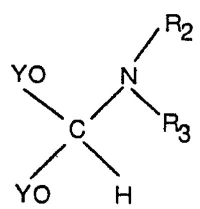
(VI)

worin $R_2$ und $R_3$ die in Anspruch 1 angegebene Bedeutung besitzen und Y eine Methyl- oder Ethylgruppe bedeutet, mit einem Amin der in Anspruch 20 angegebenen Formel (II) umgesetzt wird.
25. Ein Verfahren nach Anspruch 24, dadurch gekennzeichnet, daß die Umsetzung bei einer Temperatur von 20 bis 80°C vorgenommen wird.
26. Verfahren zum Herstellen von Verbindungen der allgemeinen Formel (I) der in Anspruch 1 angegebenen Art, bei welchem eine Verbindung der allgemeinen Formel (VII a) oder (VII b)

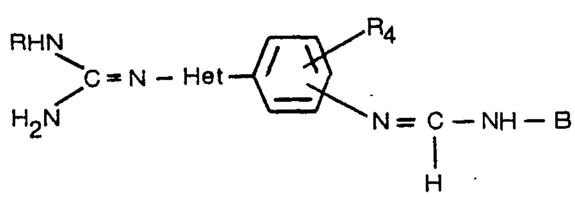
(VIIa)

oder 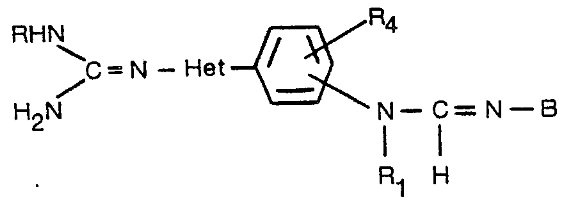 (VIIb)

worin R, $R_1$, $R_4$ und Het die in Anspruch 1 angegebene Bedeutung besitzen und B für eine Cyano-, Acetyl, Carbethoxy-oder Carbamylgruppe steht, mit einem Amin der allgemeinen Formel (IV)

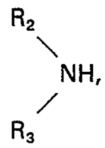 (IV)

worin $R_2$ und $R_3$ die in Anspruch 1 angegebene Bedeutung besitzen, umgesetzt wird.

27. Ein Verfahren nach Anspruch 26, dadurch gekennzeichnet, daß die Umsetzung in Anwesenheit von Wasser oder von Dimethylformamid vorgenommen wird.

28. Ein Verfahren nach den Ansprüchen 26 und 27, dadurch gekennzeichnet, daß die Umsetzung bei Raumtemperatur vorgenommen wird.

29. Pharmazeutische Zusammensetzungen, die als aktive Komponente zumindest eine Verbindung der allgemeinen Formel (I) nach Anspruch 1 oder Tautomere oder ein physiologisch verträgliches Säureadditionssalz davon in Verbindung mit einem pharmazeutischen Träger oder einen Arzneimittelträger enthalten.

30. Pharmazeutische Zusammensetzungen nach Anspruch 29, für die Verwendung gegen Magengeschwüre.

31. Pharmazeutische Zusammensetzungen nach Anspruch 29 für die Behandlung von Patienten, die unter Störungen des Magen-Darmtraktes leiden.

32. Neue Zwischenprodukte der Formeln (VII a) und (VII b)

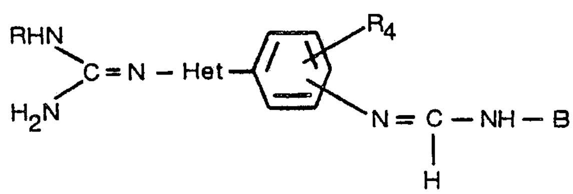 (VIIa)

und

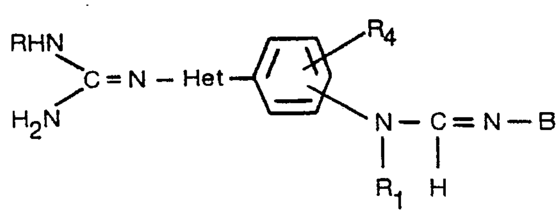 (VIIb)

worin R, $R_1$, $R_4$ und Het die in Anspruch 1 angegebene Bedeutung besitzen und B, wie in Anspruch 26 für die Durchführung des Verfahrens nach Anspruch 26 definiert ist.

**Patentansprüche für den Vertragsstaat AT**

1. Verfahren zum Herstellen von Verbindungen der allgemeinen Formel

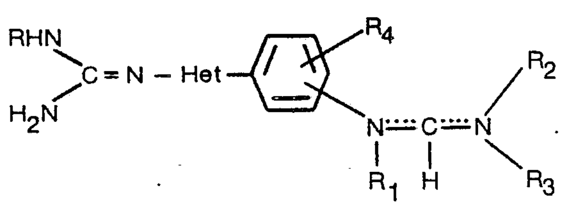 (I)

worin die untereinander gleichen oder voneinander verschiedenen Reste R, $R_1$ und $R_2$ je für Wasserstoff oder eine Alkylgruppe mit 1 bis 4 C-Atomen stehen, $R_3$ eine geradkettige oder verzweigtkettige und gegebenenfalls Heteroatome wie Sauerstoff, Schwefel oder Stickstoff enthaltende Alkylgruppe, eine geradkettige oder verzweigtkettige $C_3$—$C_5$ Alkenylgruppe, eine $C_3$—$C_4$ Alkinylgruppe, eine Cyanogruppe, eine $C_3$—$C_6$ Cycloalkyl- oder cycloaliphatische Alkylgruppe, eine Norbornylgruppe, eine Phenylgruppe, ein

ungesättigter, sechsgliedriger heterocyclischer Ring; oder $R_3$ und $R_2$ gemeinsam mit dam angrenzenden Stickstoffatom einen gesättigten fünf- oder sechsgliedrigen heterocyclischen Ring bilden, der ein weiteres Heteroatom enthalten kann, $R_4$ Wasserstoff, ein Alkyl- oder Alkoxygruppe mit 1 bis 4 C-Atomen oder ein Halogenatom bedeutet und Het einen substituierten oder unsubstituierten fünfgliedrigen, heterocyclischen Ring mit zwei oder drei Heteroatomen bedeutet und Tautomere und Säureadditionssalze hievon, die gepunktete Linie bedeutet, daß eine Doppelbindung zwischen dem C-Atom und einem der Stickstoffatome existiert und daß entweder $R_1$ oder $R_2$ nicht vorhanden ist, dadurch gekennzeichnet, daß durch Umsetzung einer Verbindung der allgemeinen Formel (II)

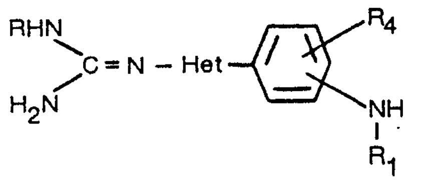

(II)

worin R, $R_1$, $R_4$ und Het die oben angegebenen Bedeutungen besitzen, mit einer Verbindung der allgemeinen Formel (III)

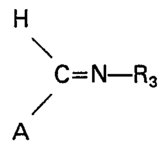

(III)

in welcher $R_3$ die ioben angegebene Bedeutung besitzt und A eine Methoxy- oder Ethoxygruppe darstellt, umgesetzt wird.

2. Verfahren nach Anspruch 1, dadurch gekennzeichnet, daß das die Umsetzung in Anwesenheit eines inerten organischen Lösungsmittels vorgenommen wird.

3. Verfahren nach Anspruch 2, dadurch gekennzeichnet, daß das inerte organische Lösungsmittel Ethanol, Dioxan oder Aceton ist.

4. Verfahren nach den Ansprüchen 1 bis 3, dadurch gekennzeichnet, daß die Umsetzung bei einer Temperatur von 20 bis 60°C vorgenommen wird.

5. Verfahren zum Herstellen von Verbindungen der allgemeinen Formel (I) der in Anspruch 1 beanspruchten Art, dadurch gekennzeichnet, daß eine Verbindung der allgemeinen Formel (VI)

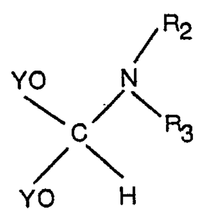

(VI)

worin $R_2$ und $R_3$ die in Anspruch 1 angegebene Bedeutung besitzen und Y eine Methyl- oder Ethylgruppe bedeutet, mit einem Amin der in Anspruch 1 angegebenen Formel (II) umgesetzt wird.

6. Verfahren nach Anspruch 5, dadurch gekennzeichnet, daß die Umsetzung bei einer Temperatur von 20 bis 80°C vorgenommen wird.

7. Verfahren zum Herstellen von Verbindungen der allgemeinen Formel (I) der in Anspruch 1 angegebenen Art, dadurch gekennzeichnet, daß eine Verbindung der allgemeinen Formel (VII a) oder (VII b)

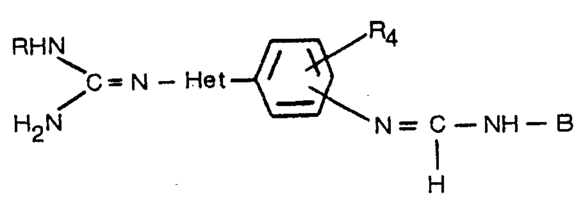

(VIIa)

oder

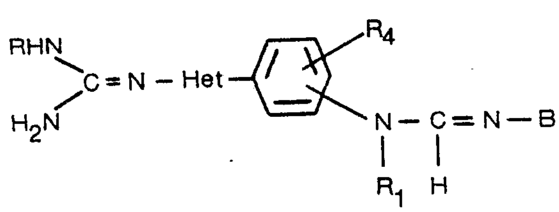

(VIIb)

worin R, $R_1$, $R_4$ und Het die in Anspruch 1 angegebene Bedeutung besitzen und B für eine Cyano-, Acetyl-, Carbethoxy-oder Carbamylgruppe steht, mit einem Amin der allgemeinen Formel (IV)

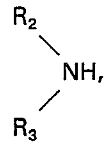
(IV)

worin $R_2$ und $R_3$ dit in Anspruch 1 angegebene Bedeutung besitzen, umgesetzt wird.

8. Verfahren nach Anspruch 7, dadurch gekennzeichnet, daß die Umsetzung in Anwesenheit von Wasser oder von Dimethylformamid vorgenommen wird.

9. Verfahren nach Anspruch 7 und 8, dadurch gekennzeichnet, daß die Umsetzung bei Raumtemperatur vorgenommen wird.

**Revendications pour les Etats contractants: BE CH DE FR IT LI LU NL SE**

1. Composé de formule générale (I)

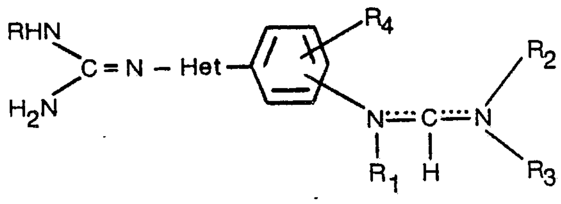
(I)

dans laquelle R, $R_1$ et $R_2$, qui peuvent être identiques ou différents, représentent chacun un atome d'hydrogène ou un groupe alkyle ayant de 1 à 4 atomes de carbone, $R_3$ représente un groupe alkyle $C_1$—$C_8$ linéaire ou rampfié contenant éventuellement des hétéroatomes comme un atome d'oxygène, de soufre ou d'azote, un groupe alkényle $C_3$—$C_5$ linéaire ou ramifié, un groupe alkylnyle $C_3$—$C_4$, un groupe cyano, un groupe cycloalkyle ou alkyl-cycloaliphatique $C_3$—$C_6$, un groupe norbornyle, un groupe phényle, un anneau hétérocyclique à six éléments insaturé; ou $R_3$ et $R_2$ ensemble avec l'atome d'azote adjacent représentent un anneau hétérocyclique saturé à cinq ou six éléments qui peut contenir un hétéroatome supplémentaire, $R_4$ represénte un atome d'hydrogène, un groupe alkyle ou alkoxy ayant tous deux 1 à 4 atomes de carbone, ou un atome d'halogène, Het représente un anneau hétérocyclique à cinq éléments substitué ou insubstitué contenant deux ou trois hétéroatomes, et ses tautomères et les sels d'addition acides des composés précités, la ligne en pointillé indiquant qu'il existe une double liaison entre l'atome C et l'un des atomes N et que soit $R_1$, soit $R_2$ ne sont pas présents respectivement.

2. Sels d'addition acides compatibles physiologiquement des composés de formule (I) tels que revendiqués dans la revendication 1.

3. Sels selon la revendication 2 formés avec l'acide chlorhydrique, l'acide sulfurique, l'acide maléique ou l'acide fumarique.

4. Composés selon l'une quelconque des revendications précédentes dans lesquels le radical amidine est en position méta de l'anneau benzénique par rapport au groupe Het.

5. Composés selon l'une quelconque des revendications précédentes dans lesquels R, $R_1$ et $R_4$ sont des atomes d'hydrogène, $R_2$ est absent, $R_3$ est un groupe méthyle, éthyle, n-propyle, isopropyle, n-butyle, isobutyle, sec-butyle, néopentyle, n-hexyle, n-octyle, allyle, di-méthyl-allyle, propargyle, cyclopropylméthyle, cyclohexyle, norbornyle, phényle, pyridile, hydroxypropyle, méthoxyéthyle, méthylthioéthyle ou cyanoéthyle, et Het est un groupe thiazolyle ou 1,2,4-thiadiazolyle.

6. N-méthyl-N'-[3-(2-guanidino-4-thiazolyl) phényl] formamidine.

7. N-éthyl-N'-[3-(2-guanidino-4-thiazolyl) phényl] formamidine.

8. N-propyl-N'-[3-(2-guanidino-4-thiazolyl) phényl] formamidine.

9. N-isopropyl-N'-[3-(2-guanidino-4-thiazolyl) phényl] formamidine.

10. N-butyl-N'-[3-(2-guanidino-4-thiazolyl) phényl] formamidine.

11. N-sec-butyl-N'-[3-(2-guanidino-4-thiazolyl) phényl] formamidine.

12. N-isobutyl-N'-[3-(2-guanidino-4-thiazolyl) phényl] formamidine.

13. N-allyl-N'-[3-(2-guanidino-4-thiazolyl) phényl] formamidine.

14. N-propargyl-N'-[3-(2-guanidino-4-thiazolyl) phényl] formamidine.

15. N-(2-méthoxyéthyl)-N'-[3-(2-guanidino-4-thiazolyl) phényl] formamidine.

16. N-(3-hydroxypropyl)-N'-[3-(2-guanidino-4-thiazolyl) phényl] formamidine.

17. N-éthyl-N'-[3-(5-guanidino-1,2,4-thiadiazol-3-yl)phenyl]formamidine.

18. Sels d'addition acides compatibles physiologiquement des composés tels que revendiqués dans les revendications 6 à 17.

19. Sels d'addition formés avec l'acide chlorhydrique, sulfurique, maléique ou fumarique des composés revendiqués dans les revendications 6 à 17.

20. Procédé pour la préparation des composés de formule générale (I) de la revendication 1 consistant en ce qu'on fait réagir un composé de formule (II)

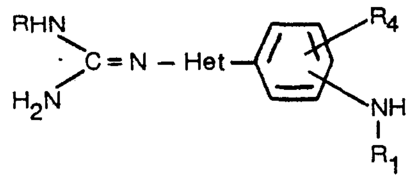

(II)

(dans laquelle R, $R_1$, $R_4$ et Het sont tels que définis à la revendication 1) avec un composé de formule (III)

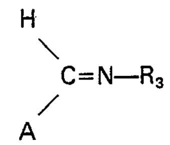

(III)

dans laquelle $R_3$ est tel que défini à la revendication 1 et A représente un groupe méthoxy ou éthoxy.

21. Procédé selon la revendication 20 selon lequel la réaction est exécutée en présence d'un solvant organique inerte.

22. Procédé selon la revendication 21 selon lequel le solvant organique inerte est l'éthanol, le dioxane ou l'acétone.

23. Procédé selon les revendications 20 à 22 selon lequel la réaction est exécutée à une température de 20 à 60°C.

24. Procédé pour la préparation des composés de formule générale (I) de la revendication 1 consistant en ce qu'on fait réagir un composé de formule (VI)

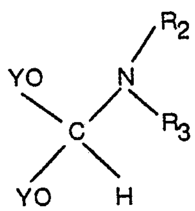

(VI)

dans laquelle $R_2$, $R_3$ sont tels que définis à la revendication 1 et Y représente un groupe méthyle ou éthyle, avec une amine de formule (II) telle que définie à la revendication 20.

25. Procédé selon la revendication 24 selon lequel la réaction est exécutée à une température de 20 à 80°C.

26. Procédé pour la préparation des composés de formule générale (I) selon la revendication 1 consistant en ce qu'on fait réagir un composé de formule (VIIa) ou (VIIb)

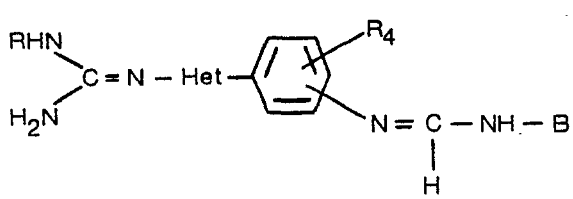

(VIIa)

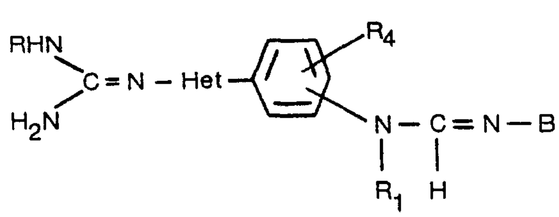

(VIIb)

dans lesquelles R, $R_1$, $R_4$ et Het sont tels que définis à la revendication 1 et B représente un groupe cyano, acétyle, carbéthoxy ou carbamyle, avec une amine de formule (IV)

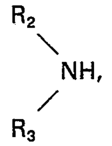

(IV)

(dans laquelle $R_2$ et $R_3$ sont tels que définis à la revendication 1).

27. Procédé selon la revendication 26 selon lequel la réaction est exécutée en présence d'eau ou de diméthylformamide.

28. Procédé selon les revendications 26 et 27 selon lequel la réaction est effectuée à la température de la pièce.

29. Compositions pharmaceutiques comprenant comme ingrédient actif ou moins un composé de formule générale (I) défini à la revendication 1 ou un tautomère ou un sel d'addition acide compatible physiologiquement de ce composé, en association avec un véhicule ou un excipient pharmaceutique.

30. Compositions pharmaceutiques de la revendication 29 destinées à être utilisées contre les ulcères.

31. Compositions pharmaceutiques de la revendication 29 pour le traitement de patients souffrant de désordre du tractus gastrointestinal.

32. Nouveaux composés intermédiaires de formule (VIIa) et (VIIb)

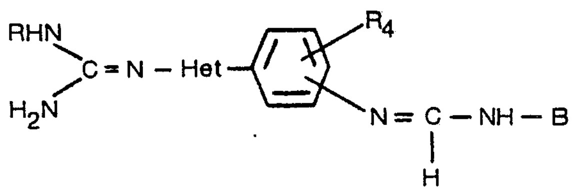

(VIIa)

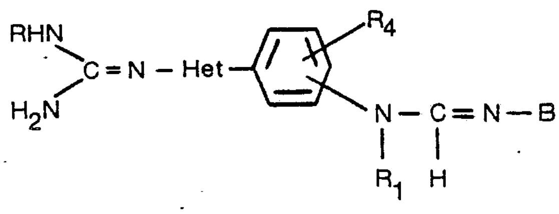

(VIIb)

dans lesquelles R, $R_1$, $R_4$ et Het sont tels que définis à la revendication 1 et B est tel que défini à la revendication 26 pour la mise en oeuvre du procédé de la revendication 26.

**Revendications pour l'Etat contractant: AT**

1. Procédé pour la préparation de composès de formule générale (I)

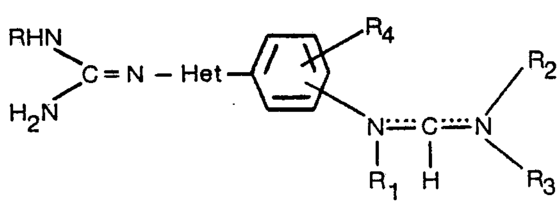

(I)

dans laquelle R, $R_1$ et $R_2$, qui peuvent être identiques ou différents, représentent chacun un atome d'hydrogène ou un groupe alkyle ayant de 1 à 4 atomes de carbone, $R_3$ représente un groupe alkyle $C_1$—$C_8$ linéaire ou rampfié contenant éventuellement des hétéroatomes comme un atome d'oxygène, de soufre ou d'azote, un groupe alkényle $C_3$—$C_5$ linéaire ou ramifié, un groupe alkylnyle $C_3$—$C_4$, un groupe cyano, un groupe cycloalkyle ou alkyl-cycloaliphatique $C_3$—$C_6$, un groupe norbornyle, un groupe phényle, un anneau hétérocyclique à six éléments insaturé; ou $R_3$ et $R_2$ ensemble avec l'atome d'azote adjacent représentent un anneau hétérocyclique saturé à cinq ou six éléments qui peut contenir un hétéroatome supplémentaire, $R_4$ représente un atome d'hydrogène, un groupe alkyle ou alkoxy ayant tous deux 1 à 4 atomes de carbone, ou un atome d'halogène, Het représente un anneau hétérocyclique à cinq éléments substitué ou insubstitué contenant deux ou trois hétéroatomes, et ses tautomères et les sels d'addition acides des composés précités, la ligne en pointillé indiquant qu'il existe une double liaison entre l'atome C et l'un des atomes N et que soit $R_1$, soit $R_2$ ne sont pas présents respectivement, caractérisé en ce qu'on fait réagir un composé de formule (II)

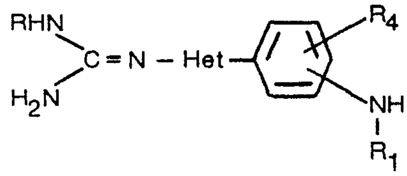

(II)

(dans laquelle R, $R_1$, $R_4$ et Het sont tels que définis plus haut) avec un composé de formule (III)

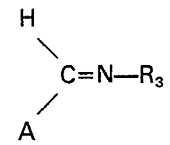

(III)

dans laquelle $R_3$ est tel que défini plus haut et A représente un groupe méthoxy ou éthoxy.

2. Procédé selon la revendication 1 selon lequel la réaction est effectuée en présence d'un solvant organique inerte.

3. Procédé selon la revendication 2 selon lequel le solvant organique inerte est l'éthanol, le dioxane ou l'acétone.

4. Procédé selon les revendications 1 à 3 selon lequel la réaction est exécutée à une température de 20 à 60°C.

5. Procédé pour la préparation de composés de formule générale (I) selon la revendication 1 qui consiste en ce qu'on fait réagir un composé de formule (VI)

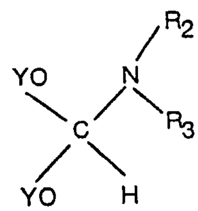

(VI)

dans laquelle $R_2$, $R_3$ sont tels que définis à la revendication 1 et Y représente un groupe méthyle ou éthyle, avec une amine de formule (II) telle que définie à la revendication 1.

6. Procédé selon la revendication 5 selon lequel la réaction est effectuée à une température de 20 à 80°C.

7. Procédé pour la préparation des composés de formule générale (I) de la revendication 1 consistant en ce qu'on fait réagir un composé de formule (VII a) ou (VII b)

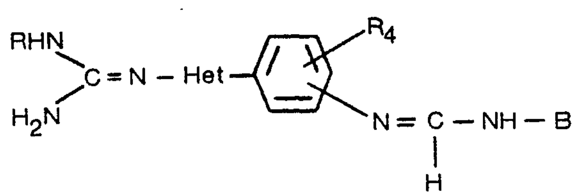

(VIIa)

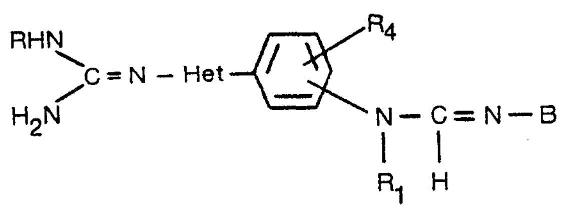

(VIIb)

dans lesquelles R, $R_1$, $R_4$ et Het sont tels que définis à la revendication 1 et B représente un groupe cyano, acétyle, carbéthoxy ou carbamyle, avec une amine de formule (IV)

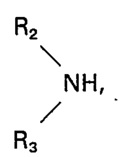

(IV)

(dans laquelle $R_2$ et $R_3$ sont tels que définis à la revendication 1).

8. Procédé selon la revendication 7 selon lequel la réaction est exécutée en présence d'eau ou de diméthylformamide.

9. Procédé selon les revendications 7 et 8 selon lequel la réaction est effectuée à la température de la pièce.